# EUROPEAN PATENT APPLICATION

(11) **EP 2 031 073 A1**
(43) Date of publication of application: **04.03.2009**
(21) Application number: 07291052.4
(22) Date of filing: 31.08.2007
(51) Int. Cl.: C12Q 1/68

(54) **Diagnostic of immune graft tolerance using TMTC3 gene expression levels**

(71) Applicant: TC LAND Expression, 44200 NANTES (FR); INSTITUT NATIONAL DE LA SANTE ET DE LA RECHERCHE MEDICALE (INSERM), 75013 Paris (FR)
(72) Inventor: Brouard, Sophie, 44240 Suce sur Erdre (FR); Giral, Magali, 44470 Carquefou (FR); Soulillou, Jean-Paul, 44100 Nantes (FR); Racape, Maud, 44300 Nantes (FR); Ashton-Chess, Joanna, 44000 Nantes (FR)
(74) Representative: Warcoin, Jacques

(57) **Abstract**

The present invention concerns a method for the in vitro diagnosis of a graft tolerant or graft non-tolerant phenotype, comprising: determining from a grafted subject biological sample an expression profile comprising TMTC3 gene, optionally measuring other parameters, and determining the presence of a graft tolerant or graft non-tolerant phenotype from said expression profile and optional other parameters, wherein said method does not comprise determining an expression profile comprising, in addition to TMTC3, the following 7 genes: BUB1B, CDC2, CHEK1, MS4A1, RAB30, RHOH, and SYNGR3. Said method may further comprise, if said subject is diagnosed as a graft non-tolerant subject, diagnosing from the expression profile if said subject is developing chronic rejection.

SEQUENCE LISTING

<110> TCLAND R
      INSTITUT NATIONAL DE LA SANTE ET DE LA RECHERCHE MEDICALE
      (INSERM)

<120> Diagnostic of immune graft tolerance using TMTC3 gene
      expression levels

<130> D25859

<160> 3

<170> PatentIn version 3.3


<210> 1
<211> 55360
<212> DNA
<213> Homo sapiens

<400> 1

ggggcggcgg ggaaggaccg agaggcggga ggagcagcgg ctcaggcgcc tgcaaactgg      60
tggcctgaac gaggtagacc atgactgtgg tttcagtggc gtcactcgct gggctgctct     120
tcctgaggtt ttcctaagcc atcccctggc ggaaccgccc ccaggtgagg tcgagctgtc     180
ggtcagcata ttgggaggaa ttgaggggag cagtcctggg ttagagaagc ttctgctatt     240
ggggccgtag ggcctggtcc cattctgaaa aagtgggaac accctgcctt ctcttcaggc     300
gtttgcaagc ggcgtccagg gtagtgcctt ctgtaagaag gaaaaaacaa aaagttttag     360
ccttgtttag tatcagaatc tgtcttgata gttagagtga gagccgcgca gccgtgatcc     420
tcgtttgggg gcgagcgaag aattgacaag aggtctcggc atcccgaccc cgcccgcctt     480
acctgtagca ccattccttt cgcggatcgt ggggcttgat ggttgtggag ctagtataaa     540
gtcaaggctc tagagaggag aggttagtac ttgtccggac cactgccggg ggtggagaga     600
gaagggatcc aaggactcac ctgtactatt gttgccactt gcttggttac ctagggtgcc     660
cttccatgaa agcgccttct ctggtgttta ccactcttac tcgtgcaagt tttgctcatc     720
ctgctcgtct gctcacaatc atgctggagt atgatacatc tctacctaac tttcctaaga     780
agctgtagac ttaaatctaa atgacatttc caactatctg gaccagtgct ttgtcttttt     840
ttttttttaa acgctgcttt aaaaaaaaaa ttactgtact tgacagtaaa ttttcctttc     900
gaaaataagt aatgagtttc tttttaaacc cccaatttgg gtaaaataag tagctcagta     960
caaaataaaa agatactgat tttgtgagat gaattttaaa gacattagtt agatttatag    1020
gaatgacaat tttactactt ttctgaaaga ttatatgcca aatactattc attatcttgt    1080
ttattcttcc ttacaatcca gtgaaatagg tgcttacatt gtcctcattt tccatctgag    1140
ttaagaaact gaggcttgga gaagctaagt aacttaaata gtgtgattga acacagatct    1200
ctctggctgg aggacatatt cttaacttct atacacctcc tcagacattc tgggaaacca    1260
atatttgtga agtgcttgtt cctcaatctt gctattttta ttaaatgtta tattttaatt    1320
ttgtaagtgt ttactcaata atttgaatta aacattcttc atgttcagtg ttcttaattc    1380
ataatttaac atgggatggt gtttctggaa aggtgttctg atctggaccc caggagaggg    1440
ctcctggatc tcgccaagaa agaattggag gtgcatacac ccccattgga ggtggtagac    1500
taaggattgt tatagttatt tcttgattat atgctaaaca aggggtgggt tattcatgag    1560
ttttctggga agagggtagg ccattcctag atctgagtgt tcctcccctt tttagactat    1620
atagagtaac ttcttcacat tgccatggca tctgtaaact gtcatagcac tggtgggagt    1680
gtcttttagg atgctaatgc attataatta gtgtataatg agcagtgaga acgagcagac    1740
gtcccacctt tctcgccatc ttggatttgg tgggatttgg ctggcttctt tactgcatgc    1800
tgttttatca gcaaagtctt tatgatcagt atcttgtgcc cacctcctat ctcatcctgt    1860
gacttagaat gactgacaga catcctgaga atgcagccca gtaggtctca cccttatttt    1920
acccagcccc tattcaagat ggagttgttg tgattcaaat acctctgaca atggtaggaa    1980
tgaaatttaa tagttactga gcacatgtta tgtcatacac tatgctgact actgcgtgtt    2040
tgttttcata atagaagtct gttttctcta acaccttttc ttctgttttt tttttcattc    2100
gttataactt atttttatgt attcacttac aagtttcgtt tgtctgtatt actcttttct    2160
acccttctgc caccaccctc ttgctcacat tcataaactc aaggtgcctg gtctctaatg    2220
gtaaatttta ttctaatttt tcagaagtga gaacaagttc atagaagtta agtaataatt    2280

gatgaagcta agattgtaac ctagatctca gtgacaattg taactaaaag caaaatctta 2340
cctaaatttt attctttaac tcattctaac agtatttttt atcctgtttc ctgtgctagg 2400
cactggggat acagcagtga acaagtcagc tctggcttga acaagtcaac taagttttta 2460
tgcattgtcg tctgtaccta agtgtgagtc aggaaattta actcaggttc tagttcaata 2520
actcactgta tatttttgag gaaaattggc aaaactatta tttttcgtca gattttctgc 2580
acctgtcagt tttcatcaag aaatgggact tatgtgacaa tgttgtaagt ggccttgtta 2640
taagaacact tgtctggacc tgccttcttt ctgccctagg cttttgtgaa cagttttatc 2700
tgaccaaaat cacttcacaa gaaggcctca ccttttatta ttgcttacct ttgttctctg 2760
tagcaatgaa tttatattcc ttggatgtta cttctagttc ctttttgctg acttaccaag 2820
aaaagtcata gtagcttact tctccgatct ctaaaacaaa gcatggccat tttgtatcca 2880
ttgtgtgtcc taaacatttt gttttgcctt atcttcatgg caaattaagc catggttaat 2940
ggcttaattc acactgactc cctactgatc tttccagttt cctcatgttg aaggaagaca 3000
agtatctgct tgctgcacac tcattcttgt ccatgaaatt aaggcaaaac aatttgcctt 3060
aatacaattt acagttttat tgaagggatt gtatttagtt ttgtatatct ataatgtagc 3120
tcatgagaag tctttgtgaa ccaaaaagtg tctaagacgg gtctcaatca atttagaagt 3180
ttattttgcc aaggttaggg acatgccgaa aagaaataaa cactgaatca cagaaacagt 3240
ctgtagtctc ttcctttctc taagatgatt ttgagggctt cagtatttaa aggggaaaaa 3300
tccattggaa gagaaaaagg gtagatatgg taatctatgt gttgcaagag aaaaagagca 3360
gagagggaaa tagatattat gtattcacgt ggggtggtaa atcagcactt tacgtgagat 3420
aagatgaatg tcctaaagta gctacctgtg gagatattta acatttgatg tgtagctgtc 3480
tgctaagaac aaaaggagag cagtttcttg catgactcag ctttcaccct aatttttctt 3540
tatagacata atgaattgag atcccaagtt ttattttcct ttcacatttc caattgcact 3600
tggattttgt gcacatatat agtaacaccc catttttgta agcaaacaga aatcagtcat 3660
cccatccact taaactgcat ctgtaagtct gcttctgtgc ctaggaaaaa ttagaagttc 3720
tcacaactaa caagaatagt gtgttacatg tttgcaataa tggcccccag tgagtcaggt 3780
ccccgtctgc ctatgccttt gtgtaaatct gtcccttccc tgcaacctct actttgcagg 3840
tggctcttgg aagtaaccta agatgactcc acaactgagc tcgtaacatg ctctctctcc 3900
ctttctctca tactatgctc ccttggggaa tcttaaaaat tctgttatct tggccctttt 3960
ataacttctt ccccattgat tcaggatgta ccttgcttga ataataattt ttaaaatacg 4020
cactaaatag agccttataa agagcttgtg cattgaacct tgccctcctg ttgtcctgag 4080
atcatcattt aagaagccta agataaccta ttagaggatg tgttaataag tagcagagac 4140
tagttttcaa agctgagaaa tctacccttt aaaagccatc cagccctaat caagccactg 4200
gataactata agcacatgag tgaccccagg cacaactaac agaagaacca tatacatacc 4260
tatatatttg tgtgtgtgtg tgtgtgtgta tatatatata tacatatata tgtatatata 4320
tgtaaaataa agatatattg aagaattgaa ttattttgaa tgtattcatt tttaagtact 4380
tcagatgcct aacttaacaa agaaaacttt ctcagtactt aaataactga ttaaatttag 4440
gaaataaaac ttgaaactat agcaaatccc aagttcttat aaaaattcca atactatttg 4500
tgtatttagc taattattat acttttcaac tatagttgtg aatccagaaa ctacatagtc 4560
agtagtttaa tatgtttatt atgtttaata tgtttattat acgtattatt gtatatttta 4620
aaattatagt tatgcagcta atatatcaaa gtctccagga tgtcagattc tcttaaaggt 4680
tgcagttact ctaaaaaatc agatgcataa tttaatattg tttattatgc atattattgt 4740
atatttttat atatttttaa attatttaat ttattttaaa ttatttaatt taacaataat 4800
ttaacagcta atgtatcaag tctccaggat gttagattct cttaaacatt gcaagtactc 4860
taaaaattag atacataagt tgttcccaag tataacttta aagcactgaa accacaggtt 4920
taatttactt ttttcttcta ttaattttaa actttcctgt ggttagaaca tttaaccaaa 4980
caacagtttg tcaccccaag tgaattgatg ttaccaagcc agtggcctga ggttatttat 5040
gggaagagag gtttctgacc tgtacacaaa acttgatttt tacctaccca aaggtagata 5100
tcctgagttg tttcattcgg tttttctaaa ttacaacatg tatatatagc tgtcctatat 5160
tggatcccat ttcctgattt ctgtatcttt gtttcttaat atactgtttc attttactag 5220
tgtacctttt ctcctagttt cttgaaaagg aacatcagag gtaaattttt tgagaccttt 5280
aatgacttag gtttttattc tactttccca cctggaaaac ttaggtttat taatatagaa 5340
ttttagttta gaaatcaata tgcctcagaa gtttgaagca ttactccttt tttattttgg 5400
aagggaattt tttgttttgt tttgttctta acagcatttt gggtgaaaaa aaaaagattt 5460
ttgattcctg atccttactc tggaaccttt tttatttcca gaagctttta gaatcctatt 5520
tgttttcagt gttgtaaagt tttacagaat tttgtgccca gtagaaaagg gatggagggt 5580
ctcaacctaa aatatatgga cagacttttt tttacttagc ctttattttc aatatgctgc 5640
cttttccctg ttatcagaga ccctatgttt taaataatcc acagaggaaa cctccagtct 5700
tctgccttgg aataggagag gttacttggc tttatcccgt aagagagggg gctgacataa 5760
tgtccccgtt atcagggaca ttatccagta agagatgggg ctgagtttca acaatgtgac 5820
tagcttatgc ttcttagaag tggatggcat tttagctgcg cttttctctg gagaattaat 5880
atgcaattac ttacccacct actaaaattg aacttacttg gaataaatat gttccttatt 5940

ttatcttcat gatttttttt ccagtttttg tccagaagtg cttatagaaa agatggctaa 6000
tattaaccta aaagaaataa ccttaatagt aggtgtggtt actgcctgct attggaacag 6060
cctcttttgt ggttttgttt ttgatgatgt ttcagcaata ctggataaca aagacttgca 6120
tccatctaca cctttaaaaa ctttatttca aaatgacttc tggggaaccc ctatgtctga 6180
ggtaagtaat tacttacata ttacttgtac atgtctcaga ttttgaaata ttctggtatt 6240
ttattacttc taattcttta tcaacattat catcacaaca gttacagatt tttaacccta 6300
aaatgtttat atgaaacgta acatgtttgt aataaattga acaggaagct gaggatgagt 6360
actttaaagc agtacttggt tatttctttg cctgtgtacc ttggctgctg cttttttttt 6420
tttaactttt attttagata cagggctaca tatgcagatt tgtcacgtgg gtatattgcg 6480
ttatgctgat gtttggggta ccaatctgtc acccagacag cgagtatagt acccaatagg 6540
tagttttggc ttaacttctt aaaaataata tatatttatg tgaaaaccaa atgtataggg 6600
agagcaatag gtgtaataca tgcctgtttc ttgctaattg actttatttc tcttttttct 6660
gtcttgtgag taaggcaaaa gagatgtgat atatttcaac attttaaaat atggcaactt 6720
caatataaat agataagaat ttaagagtaa aaatagtaag tacacatatt gccaatttct 6780
cttgcagaag aagtggttat tacaaatttt gaatctttgt cctatctctt acataatagt 6840
tatttaggtt atatatcgag aacccaaaag tttagttcaa taatttaata ttttattcat 6900
ccatatggtt catttttatg agggtatttt tactgttttg aaggtcactg ttaaactgtt 6960
gggattaaac ccttatcagc ttgatatcat atagaatgga ttctaatttt agttatgtca 7020
tatattaact gtgtgaaata ggtgagaata catctttcag atggaaagta attttcctga 7080
tctgtaaaag aggataattc ctttccaaaa taaaaaagag agtaatgctt caaatttctg 7140
ggggatattg atttctaaac taaaattcta tattaataaa cctaaatttc ccaggtggaa 7200
aagtagaata aaaacataag tcatttaagg tctcaagtaa taaatgaatt attttatcta 7260
cactacctgg cacatagata ttgaataaat tgttgaaaca taacatgtaa ctctctgcaa 7320
tgttaacagt atgtttctcg gtcattgtgg cctctttccc cattccccaa atttggaagc 7380
ctctcaaggt taggatggaa atctttcatt cctctgatca caagcacata taattatttt 7440
gtcttattta gtaagaagac tttattcgcc tttttgttct cacccaattc tagcttttga 7500
aaaaaaatct tttgatagta agggggaaaa tatggcccta ttatacatta ttctacacag 7560
cttgtcctcc tctataccta cctttgatct ccagagaccc tcagaactat gtgtccattc 7620
cctcaattta tgctagtatc tgttatgatt ttaatcataa tgttgaggtt ttgaactgat 7680
ggtttagctg gccatattaa atggtctcat tttctagatt aggctattct tgtattgcta 7740
cgaagaaata cctgagacag ggtaatatat aagaaaagag gttttattgg ctcacagttc 7800
tgtcagctgt acaagaagca tagcagcatc tgcttctggg gaggcctcaa gaagcttgca 7860
gtcatggcag aaggggaagc aggagcaggc acttcacata gcaaaagcag gagcaagagg 7920
agtacggtgg gaggtgctac acagttttga acagccagct ctctcaagaa ctcattgtag 7980
caaatacagt accaagtggg gtggtgctaa accattcata ggaaatccac cctaataatc 8040
caatcatttc ccaccaggcc ccacttccaa tattggggat tacattttga tataagattt 8100
gggcagggac acacacatat cacataccat ggtgtcagtg atctgttcag tgattttctc 8160
agctttgcag attgaaaatt tgattttgtt cagaaagccc gtaagcagat ggactaagaa 8220
aaagactcat atgttctgac agaatggatg aacaaactag aataacagtt tattaagagt 8280
gaagagtgta gttgtgtagt ttccttgtat tttctgatgg gcatattctc tccctgaagg 8340
caaatgtgac actagggaaa attattctag gtaatcctta ggtgtcatag gtttcatgat 8400
aagcctggaa ttagcatatt caaagatcta atcattgata tcatcaagtt atgatagcct 8460
agaagatacg tgtccacacg tatcttacaa gaatttttgt accaatgcag gtcttaaaga 8520
cacattttgg gcttaggaca ttctcctaag ccatataaat actgccgtat taggggtctt 8580
tggactcatg tgaacctctc agtgtttaga ttatctctct agtaggctta tgctatagat 8640
atgtgctgct ctatttattg ataattcatt ggatctcaac cctgactgcc gtattagaat 8700
ttgcctgggg catttaaaag aatgaaaaca tattaccaag gctctgtcca aacctgttat 8760
ttcagataat ctcagagtgg gatataggta tcaatatttt tgtcttattt tgtgtgtgtg 8820
tgtttagttg ttttgtgtgg gagggagggt ggggcttttg tttaagtcat ccaggtgatt 8880
ctttggtata gccgtggctg agagcgaggg ggctgtaatg agccaccttc ctctggcatt 8940
tggcctataa agtaccttgt gctaaggtaa cttctgcaaa tattttatct cattatgatt 9000
ttgccatccc tatattgaga ttcacattaa cagggaactg ttagcttaat cattcattac 9060
actgttgtgt atttttattg ccaactaata tcatgagtaa tttttctttt aatatgcccg 9120
aaagtaataa tgatctgaat tgttttattc ataatggccc taggtgtttt cacaaagtaa 9180
acttagtatc taataggctg atgatgattt gtcacagtgg aatctgaaat agatccctaa 9240
tgagttatgt gaataattat gttaataatt ggtctgtctg ttagttggtc atcacctata 9300
gtatatgtat gtctaatagt cctaactgaa caattatgta tactgcaatt caaaatacat 9360
attcccttct actttctgag tacatccttt cacctagaat atatgttcca ttgtagccac 9420
agtgacattt caggtactcg aagggtttct agagtccttt tctactttct agtacaaatc 9480
atgtcagttc agtacagtgc cttaaaaata gctttatatg cctgagatga aatttgaagg 9540
atgtaaagga attaggcaag caggaaagaa cattctaagt agatagtaca ttatggagta 9600

tatagtcagt acagagatac taaaatgttc ctaatttcaa gatgagaaag atttattctt 9660
tgtgtttcct aatgttccct actaaggatg aacaggctta agtctacttg tgtgccagaa 9720
agttacagca aaatatgata ggaagtagaa gagcaaagaa gctgaatatg gatgcgtata 9780
ttagtctgtt ttgcgttagt ataaaggaat acctggtaat ttataaggaa aatatgttta 9840
tttggcatat ggatctgcac actgtacaag catggcaaca gcgtcttctt ggcttctggt 9900
gaggccttga aagttttact acctgtagaa ggtgaagggg gagcaggtgt atcacatggt 9960
gagaggggga gcaagaggga ggagaggagg ttccaggctc ttttaagcaa ccagctttca 10020
catgaactca ttacagtggg gaagacacca aaccattcat aaaggaccca accccaatga 10080
cccaaacatc tcccaccagg cccaaccttc aacatttcaa cctcctcata tttcagcatg 10140
agatttggag gggacaaaca tccaaactat gtcaatgccc tttgtataaa actacaaaaa 10200
ataactttat taatatttta tatatattcg aactgtgtgt tttattctta aatctggttt 10260
ggttttgtgt tagggaaaga agtatttctg attaaatgta acttataatt taaaaagaga 10320
ttcagtactt tacatgaaat tttatttatt accaaaggag aataccacaa gaatggtgtc 10380
tatttgcata ttctaagaaa gtttttactc ttgtattact ctctgtaatt tgggaattcc 10440
tagtttaata tattcagatt tacacattat ggagatgttt ttcataaaac agctagacag 10500
tacctttttg agtcatcact attttaattg aactggtaag cattctgttc ttacatttcc 10560
ttttgaagat ctagccagta gttacatgaa cttttgtatt gctgatagat atggaggcag 10620
gctttggaga gaatcctgtg ctctaacttg accaatgccg gcattgtctt tttttaaaat 10680
tgttatattg aagaggaatt aattgtcaaa tgtcctttta ctgttagttc tctgaccact 10740
gtatatccag gaggaaatta accttgtttg tggcctaagg aaatagaaaa acacagatta 10800
taaaactgta ttttattttt gtgttactct tacgacattt catattttat tgttatttct 10860
cagtaaaatg tagatattaa tatctttaat ggtaaatgca gtgatgagct tttgtaccct 10920
gttggaccaa ggtactttaa taatcactga tcgttttttc cttgtaggag agaagccaca 10980
agtcttaccg tcccttaaca gtattgacat ttcgcttaaa ttatttgtta agtgaactaa 11040
aaccaatgtc atatcatctc ctgaatatga tttttcatgc tgtggttagt gtgatatttc 11100
tcaaagtatg caaacttttt ctggacaaca agagtagtgt gattgcttct ttactttttg 11160
cagtgcaccc aatacataca gaagcagtaa gtaaaactat gaactgactt tttttctttt 11220
tcctttttta caaatcctgc acagtgatta taatggtata tatttttaag aaaaaaaaaa 11280
attaaccctg actttaatcc aaatatttga agactcttaa attcgtacag ttaattctta 11340
acttcctagt atgcaaagta taacttccta ctgtgtaaaa tttttaaatt taacattttt 11400
gaaacataaa catttttcat tcaataatat aaaatctgtt tagaaatata tacatgaata 11460
cagttcttcg gtcacttttt ttaatgaaca agatttctaa ggactattta aagtttattg 11520
tctttttaaa ctttattttt cagatttatt atgagatgta actttttgaa tttatggtgc 11580
ctttgtatat taagaacatt cgtattttaa gaaaatatgt aagaagtact taagttctag 11640
atattctacc atatttacgt ggttaccatt ggagaaagct ggataaagag tacgcaagat 11700
ctctctttac tagttttgaa acttcctata agtcttatta ttatttcaaa ataaatttaa 11760
aaaagtataa actgttaaga gtcactaata aatgtctatt tatgtaagat tcacaaattc 11820
accacctgag ggcacccaaa gaatgtttaa gaaacaaatg tgcacttgaa aagttacgga 11880
aattaaagta tttttaccat tatatatagt aatttttcaa gatattcttt attaatatga 11940
acccccttca tttttccttt ctaggtaaca ggagttgttg gaagagcaga acttttgtca 12000
tctatctttt ttctagcagc ttttttgtca tataccagat caaaaggacc agacaattcc 12060
ataagtacat gtctcacatt tgattttttt ttaatagtgc taaaacttga ttaaggaatt 12120
tttatttact ccaagtgctt cttataacac attatatacc acatttggga tttatcttta 12180
tgtattgttt tactttagca tatgtgaaaa tatgaaatat gtaacattgc tagtatttag 12240
gaagtttgtg tataatttat atttttatct tgagctaata gattttatat gatctaattt 12300
taaaaaccaa ataaccttag aatagaagag ttttagagtt agtcctagtt ccatcttctg 12360
ttatctatta atgatataga ttccagtatt tcagttgtct ttggataaat aacacctggt 12420
agactgtcat attgtttagg taaggttaaa agcctcctag aatataactt ttattgtgtg 12480
ttgatcatag ctatttattg agctcctacc ttgtgaccta ccctggaaag tgcttcgaat 12540
acattagctc attactctta atgattcctg gcattatcag aaaacaagtt ctaggcaata 12600
aaaatgattt ttttcaagtg tgggcacagt gggcacaaac ctttaagttc ttggggaagg 12660
ggcagatttt ctcactgtag ttccaaggta ctcatgacaa gaagactagt tttatgtgtg 12720
gaaactgagg ttcaagaaac acatgattta gccaaacatc ttaagagtgt tctggatttg 12780
taatagtagg tttgcctttg ctgctctgca taatgccttc ttaaaacaat ttgagtggca 12840
gcatagcata gtacttaaaa gcatggacta aagagtctgg tttcaaattc tagctccact 12900
gtttatttac tagttccatt caccgtaaca catcaggcaa attaacctct ctgtgcttaa 12960
catttcttta tctgttaaat gtgcataata atagtcccct acctgtgcag tgcatcatgt 13020
agtaggaggt acttttgtaa ctaatatgga attgcagacc agtatcctct ttaagtacca 13080
tcctcaccaa agcctgaatg gtaaagttag aaaatactgt ggcctacagg attataatca 13140
ggctctcaat tacctgtcta atcatatctt ctgccattct tgccctagct gtcttcacta 13200
tagctagata gaccttcttg ctgttcccaa acaggcagag cacccacctt tatacctgca 13260

tttcccatta ttgaattttc ttcccttaga tctttatgta gtagtctcat ttatacacta 13320
ttctatatta tcaggcctca gcttaagtat ttctgtctta gaaaagcctt ccctgttcta 13380
actgaaatag cttttcacca tattatatct tttgttccaa atacttgtac ttggtgcttt 13440
ttaatgattt cttattcttt acaattctaa tatcttgctg tatttcctta agaattactt 13500
atttgggctg ggtgcagtgg ttcacgtctg taatcccagc actttgggag gctgaggtgg 13560
gcggatcaca ttgtcaggag tttgagacca gcctgggcca ttgtggtgaa atcctgtctc 13620
tactaacaat acaaaaatta gccaggcgta gtggcaggca cctgcagtcc cacctacccg 13680
gaagaatgag gcaggagaat cgcttgaacc tgggaggcgg aggtagcagt gagtcgagat 13740
cacgccaccg cactccagcc tgggagacag aatccatctc aaaacaaaaa aaaagaatta 13800
tttgaaattc ttggtctgtc tctttaagta attctgattt agtagtatat gttattatgt 13860
ttgttctctc tgttttatga ttgttgtatc cttcagatat tgagttgttt tggcttgtga 13920
gctcaagcac atgttcttct aaagttataa actactgtgc ctgttaaagg gaaaggccag 13980
ggccactctt tgttcatcct gtgagtctga agggatgaaa ggaattataa accaggatat 14040
aaagttccag gttccgcaga gctgctgtgg atcactgttg ttcctaccag tgctactagg 14100
cacactctct gttttcttta agagaatcag cattaagagg ccagtccacc atccctctgc 14160
atttaaatgg tagctctgta gaagttactg cctaggagtg attggttggt ttgtaccttt 14220
taaaaaatat ctacctctta ccccatcaag acttctgaag tgctctgctt ttacctctgg 14280
aacacccact agccattgta gctgtggatt tctgcagtaa cagagtagag agtgatctac 14340
ccaaagcagt caggagagga taaaacaaaa gttaaaaaaa aaaattccca ctcttgtctt 14400
ctgccttaaa tgtaatccct cataacactc acagaaccac acatgcttaa aataacctcg 14460
aaatgctaag gttttgtgtg tgtgtgtgtt tttttttttt tttttacaaa aagtgtctta 14520
gaaactattt ttccctctta cttacctttt tctagggttg attttggggg aaaggaccaa 14580
caggtcaagc tagttcacca tttgagcgaa acaaaaacag agtaatgttg ttttctatac 14640
ctttaacttg catttctgaa aatactcatt tctaacctga aaatacatag tttacatttt 14700
tcttttgttt attgctttct cacaatagaa cgtgaccttc cttagggtag gtaacttttt 14760
ctccattttt tgatcttcat ttcctaaaat gattcctagt ttacagtagg tgtttagtat 14820
gagtatattg aagaaataag taagtgaatg aatgtaatgt attttaaatt ctaattgaaa 14880
aaatactgaa atctaccttg gacctctctt tatcctctta accactgtga tactttgtac 14940
tcttagtaat tttacccttg aagccacagt ttccacatca tctttgtatt ccctattgta 15000
tctagtatac actggcctgt gcaaaggttt tcaagttgta attctttata ttcattttaa 15060
cttctatatt catgtcccat accccttttg taaatatggt gatccaattt cagggcttta 15120
cacttgattt tttattactt tgggtttctg cttaaatatc cacttaaatg tcatcaatat 15180
cactactcat ctttcccagt ggtactattt ttatttccct tcatcctctt tttttctcta 15240
taacacttaa acgttgtcac catttgacag agtatattat tacttgtctc tcttccctat 15300
acccttgaaa tgtagtagat acttaaaata actgtttgaa taagtgattg ctgaagatgt 15360
gtttctttta cacatgtaga gtcagtttag aagcctgtat tttgaaatgg gaatagattt 15420
aagtgtcctt aacttattta ccaggtgtac ttgttttgga caaaatcaca tgtaactttt 15480
aaccacagtg atctgattgt aaaatcaaag tgtttatata gtaatatagt cactagaggc 15540
tgaagaactg aagtgattgg tttcagtaag acatttgcat ttattactct tagttgagat 15600
ggaattcaag tttgtaaaag gtaaccattt gcttgttctt ccttgaagta gaaatacaaa 15660
ttactgagaa gctctgactt taaaatcaga agaaccatgt taaagtcttg gtctccttat 15720
tctttgttac tttttaaaaa atacctctac aattctttcc tcatcttaaa aatgggtatg 15780
attacatcca tgttcttact ccatagagtg attttaagat tcagtgaaat aatatatgcc 15840
aaactggcta tataaactgt ttaaaatatc tgctttcaat gctattattt cctatgagtt 15900
tttatgtaga catacacatc cattcatact attctctata tccttcattt gcttagctta 15960
ctctcacccc tatttatttc tacatccacc tttcccaatt tttccatggc ttctatatcc 16020
ttcaagggcc aacattaggt ccagcctgtt ttgtaaataa ccatcttctg atttcttcct 16080
ctatgaagtt cttgttgaat ttagcattgt agtgtaatat aatctattat attagtattg 16140
atgttttggc ctaatgagat tataagattt ttttttttaa ggaaagagta ttattatact 16200
tagtatctgc aatagtttca tgggtttggt ttttttcatt tatttctttg ttttcggtta 16260
ttttttcctt cagtaatttt agtctacaga gtactttgaa aacaatttca aaaatatgta 16320
tttctatatt aagcccagtg acagaagaaa atgtattcat gaattctgtt cttgtttata 16380
tttagattta agtcaattat ttgaattttt gctttagtta atagtcttaa taaaaaccga 16440
ggtcacggcc gggcacagtg gctcacgcct gtaatcccag cactttggga ggccgaggtg 16500
ggcggatcag ctgaggtcgg gagttcaaga ccagcctgac caacatggag aaatcccgtc 16560
tctactaaaa aatacaaaat tagccaggca tggtggtgca tgcctgtaat cccagctact 16620
cgggaggctg aggcaggaga atcactcaaa gctgggaggc agaggttgca gtgaactgag 16680
accgtgccat tgcactgtag cctgggcaac aagagcgaaa ctctgtctca aaaaaaaaaa 16740
aaggaaaaaa aaaaaaaaga aaaccaaact gagatcacac agtcatacta ccagatcttt 16800
ttttaaacta gaaaagaatt agagattata gtttttcata agaaaactga agcctggaaa 16860
agctgcccaa actcacaagt tcacaatgaa ctttcacagt tcattactct ttagtcaaat 16920

tactactaca tggtagacac ttacttaaag ttgatgaagt atgtttttca tttaaataga 16980
acttatagtc tattgcaaat tttttgaaga atagtattta tttaagtgcc tattgttaac 17040
cactttcatt ttagttttta taaaaatggg ctagacacaa tggctcgtgc ctggaatccc 17100
agcatggtgg gaggatggct tgaggccaag agtttgagac cagcctgagc agcatagcaa 17160
caccctgtct ctacagaaat aaaaaaaaaa atagccagat gtggtggcac atgcctgtag 17220
tcctatttgg gaggctgagg cagatggatt gcctgaactt cagaattcaa ggcagtagtg 17280
agctatgttc acaccactgt acttactcca gcctgagtga cagggccaga ccctgtctta 17340
aaaaaaaaaa aaaagtatag taccctactt ttcccaatac ttttaaattt taagttttta 17400
ataatttatc agtttaataa ggtataaaga tatctgtcat aatttcagga atttgtcttc 17460
atttcagaat gatagctcat taattatgac acacaaacct gtaaatttct cttctccaaa 17520
ttcaccaccc tacctttgtt ttaaaagtta gtatttgatg aaataatttt tctctttgta 17580
aaaaatttca gttcaaatta tacatgttga tattttgctt tgaatgtata aagtaaaaat 17640
tattaaattt aagtatattt taaagtatgt actactaatt aatgagaaat agcactcata 17700
tggatatgga atcacaaagt ttaaaatatc tttttttgat attataaaat tgttttctga 17760
atttttatat tttctgttct caaattgcag tatggactcc aattgccttg acagtgtttt 17820
tagtggctgt tgcaacatta tgtaaagaac aaggaataac agttgtagga atttgctgtg 17880
tgtatgaagt gtttattgcc cagggggtaa gccaaactat aaatatataa attttcttat 17940
tgatatattt tatcctagtt gaatcaaatt ttatttattt ttatttctta aatgtatcat 18000
tatgtaaatt aatgtgttta ccaataataa taccagaaat catgttttat cctcatacct 18060
actgaaaatt acttaacatt ttatgatact ttaagtgttt taagaactgt gttttaaatt 18120
tcaataatgg aatcattagg cagcttggaa aagtatgcaa tagtactgta cagtataatt 18180
cattattagg taagactgtt gagctcagtt ttattcatta ttagcttcac acattcttcc 18240
acattaattt tataattata cttgtatcta atcttgcaat tagaaagtac tacagtattg 18300
aaaacattga gatatgtcgt tatttgttgc ttaaaacatt tcttttcttt ttcagtatac 18360
tttgccatta ctatgtacta ctgctggaca gtttctccgt ggaaagggta gcattccatt 18420
ttctatgctg cagacactag taaaactcat tgtcttgatg ttcagtacat tattacttgt 18480
tgtgattaga gtccaggtta ttcaatccca acttccagta ttcaccaggt atgaaattct 18540
ggttctttgt tttctccatt ctttttttta actttggatt ttaatgatca taaatgttga 18600
agaaagtatt ttattttgtt ttttaacagt tttattaagc tataattaac attttttaaa 18660
ctgcatatat ttaaaattta tgtactgata atttttgaca tatgtataca cccttaaagc 18720
ccccacaata aagataatga atgacccatc ccaaactggt taagtgttaa ggttagtgtt 18780
aatttagtct cctatatcct tagtgatttt tgtctactca ttctgtcagt tactgagaga 18840
tgttgaaatc tctgactata cttgtagatt tcgctatttc tccttgcact tctgtcggtt 18900
tttgttttgt gcgtttttga agctctgcta ttaggtgcgt aaacaactag gattgtcaag 18960
tcatcttgag gaactgatcc ctttgtcatt attaaatgag cttcatccct agtaatagtc 19020
tttactcaga aattcacttt ttcttatcac agctgtcttt cgttagtgtt aggtggtgta 19080
tctttttcca tctttttact ttttacctgt ctatgctttt atttataaag aggatttctt 19140
gtagcatcca tacaatttgg tcttcttttg ttgtttggtc agataatctg acttttagat 19200
gggatatatc attgatttat atttaatttg ataattgata tagtttgatg gaaatctttc 19260
atcttgataa aattgttttc tctttgtctc atctaatctt tgttcctttt ttctctcttt 19320
ccaccttcct ttggattctt tttttaatat ttcattttat ctccttgttg gcttattagc 19380
tataaatctt tgttgtttta gtgttgcttt agggatttta gaacatcctc tgcttatcac 19440
agtttacctt caaggaatat tctaatttcg aatatatata taaaataaaa atatcagtgt 19500
atatatagta taaatatatg taatatacat tttatatatg taagaactta acttttgtgc 19560
agtttgcata tgttttcctt gaacatacat tgctttaaac aattgtcttg gaggaattta 19620
aataataaaa aagtatttta cattaaccat gagtcaccat ttccagtgtt ctttattcct 19680
ttgttggata agatttttat ctggtactat ttttcctttt acctgaagga atttctttag 19740
cacttctgat taaaaatatc aggttttgaa agataccttc tctgggtgga gaattctaca 19800
ctggcttttt cattcagtat tctgaaaatg taaatctact gtcttccggc ttttattgtt 19860
ttcagtggga aaacctctat catccttatc tctttatgct aatgtatgtt atgtgtattt 19920
tttctatgcc tgtttttaat atttcctgtt taccattgtt tcttatgtat ttatgtttct 19980
tgtgcttagg taaactgagc ttcttgaaat ctgtagattt taattttgta aaaattttgg 20040
aaaatgttta gctagtcttc aaatattttt gccatctttt ctttttccta tttttcttgt 20100
atgtcagtta agtatattta agtttcttga agttgttgca ctgatgctct gtttttttcc 20160
ctcaatcttt taccattttt agtttggata gtttcttttg ctatatcaag ttcactaatc 20220
ttttttctgc aatatctaat ctactcttaa tttcatctag ttgtttctta cctctgaatc 20280
tgttttcatc ttcagaagga agtctgattt gagtattctt ttatatcttc tatatctcta 20340
catgttctat tgctttaatg tccttgccta ctaattctat catctgtgtc attccttggt 20400
cagtttttat tgaataaatt ttctcttcat tataggttgt atttctctgc ttctttgcat 20460
gtcgggaaat tgttgaacag ataccatttg tgaattttac cttaatgtat tctataaatt 20520
ttgagtttta ttctggaatg catttaagtt acttggaaaa tgtttatctt ttcagtcttg 20580

ctttttttct ttcttctttt tttttttgag attagagttt cgcttttgtt gcccagggct 20640
ggagtgcaat ggcgcaatct cggctcaccg caacctccac ctcccaggtt taaacgattc 20700
tcctgcctca gcctactgag tagctgagac tacaggcatg cgccaccacg cccagctgat 20760
ttttgtattt ttagtagaga tagggtttct ccatattggt caggctgatc ttgaactcct 20820
gacctgaggt gatccgccca cctcagcctc ccaaagtgct gagattacag gcatgagcca 20880
ctgcacccag cccagtcttg cttttaagct ttgttttgtg ggaccacaat agcatttagg 20940
ataagactaa tttcacttca ctactgatgc aagatctttt tgactattgg atgtgaactg 21000
taaattacga agatttccac actgtctgcc gaggaactat tcctggctcg gtatatttgg 21060
gaatttttag ctctaatgca ttcaggaggt ttttgcctga ttgtattagt tttctgaagg 21120
ctgctgtaac aaataactac aaactgaata gcttaaaata acagaaattt gttccctcac 21180
agttcaagag gccagaaata ccaaatcagt atgtcagcac aattggttcc ttctggaggc 21240
tctaaggaag aatttgttcc ataactgtct gctggtttct ggtggttgct agcagtcttt 21300
ggcattcctt ggcttgtaac tccatcactt caatctactt tgtcttcaca ttgccttctt 21360
ttccagtgta tctctttctt ctctttttat gaggacccca gtcgttggat ttagggccca 21420
ccctaaccca gtgtgacctc atcttaacta atattggcag aaactatttt caaataacac 21480
cacattctga agttagggta gacatgaatt ttttagaaat atgattcaac ccactacacc 21540
gaccatgggt atttcctcac atatatgtgc tgatttgaac tcatcaaaat caggccaggt 21600
gtggtggctc acgcctgtaa tcccagcact ttgagaggct gaggtgatca cttgaggtca 21660
ggagtttgag accagcctgg ccaacatggg gaaacctcat ctctactaaa aatagaaaaa 21720
ttagccagcg tggtggtgca cgactgatcc cacctactcg ggaggctgag gcacaagaat 21780
cgtttgaacc tgggaggcag aggttgcagt gagccaagac tgtgccatgg cattccagcc 21840
tgggtgacaa agcaagactt tgtctcaaaa aaaaaaaaaa aaaaaaaaga actcatcaat 21900
attgaaaact ggagagagac cctctacaga tgtctcaggt tctctttctc agcagctttc 21960
ttctgtctgg attcttcctc actaattcta gcccccgcac tcctagggct cccaacttca 22020
tactctcaat tcagtatacc actgggctca gcctgggttc cctcttgctg ttttactgac 22080
taaaaactct atccaggtac taaactcttt ccagaaaaag ctccccttat ttgtttttct 22140
tctctcaggt attacagtcc tttttcacct tatgctcagt atgtttaaaa aaaaattata 22200
cctcttatac agttttttag ttgtttaagg ctagaaagca gaagatatag tgaattttaa 22260
gtctgcatat catatttatc tactttaaca tttagccttt tttcatttca cctgacttgg 22320
ctaaaattta aaatgaaatt tatactattt ccctgttcaa ttttaagtta ttgccattac 22380
atgcaagaac ttttattttt aacaaaaggc caacatgtta ttcacattat gttacatgat 22440
cgtgcattgc tggcgctggg tagaataaat tttattcaga taatttttaa acccaaaact 22500
gtgtgatatt ttcacataac tatcctccct agcatttttt tttctttaaa gaataattac 22560
ttgttaatat cctggatttt tatctctgag tctagccgtg ctaaaatgaa cttgtgcaga 22620
tattacagta ttggaaatag agaagattga attgtttata taagacttcg gatttccttc 22680
ttagtttcat tggttttctg ttattccttt tagagaattt gaatcaacta ttttctattt 22740
ccctatctac agtctaaaaa tatagtttat aggtgtgaat ttatgtatgt gttagtaggt 22800
atgaatatgg tgcttatagc atataaatat tttccaaata aagcatttca acattgtgta 22860
tatatgtttg tgtatgtgtg tataaattct attaccctct gaagttggat ttaatataat 22920
ttgatttatt ttgacattaa ctctttcttt aaatcagcta tcctaaataa actgtaattt 22980
ttaaaactaa attccgaata gtgaattata ggcaaaaaga taacttttta ttcagttctg 23040
ctggttattt tgagaattca agttcagact caagatttat tattttatct gaaaaataat 23100
atgtaagttg tgagattccg tatgaaaatt ttgaaatgat attatttgtt caaatagtat 23160
ttatttttat caaatatttg atatgttttc agaaaatcaa gtcaaataag tggcagtagt 23220
tgattttcct tggcttactc atcttaaaat gagaaaatgg gtcttttaag tactatatat 23280
tataaccaat ataatttaga acacagacta aactaatgaa atgtatatat gttgtggcct 23340
gggaaaagat aaactatgat ataagcttat attagtattt actaagagag aaagtagaac 23400
acaaattctt acacaggcct acacagttac tactttgtac tagaaatttg gtttttagct 23460
tcctaggagt caaagaaaag agggaaggaa gctaggtgat atggttttct ctctccatga 23520
cagaagaaaa atatacgaat tttttccagg aagcataatt tttaattagc atttagctct 23580
aaaagaaatg tctcatgtag gaaagcattg gtgagtgttt tcaaaagtga ttcatacaac 23640
acatacaacc ataaatctta tacaactttt tgttactact tttaatcaaa aggtttaatg 23700
gaacactttt gcagtgttgt aatattatag gatgcatgtc tgaattattc taataataaa 23760
cagaaatgga tgacctgctt gcctttcaaa cagtccttca taaatatcac ttctctgaag 23820
tagatgtttc ataaagtttt gatagtttga actctagcaa gagtctgagg tacacataac 23880
atttgtctgt gtcttctgtt taataagatg atatataaaa tgtttattgt ttagtgtttt 23940
acttatgtaa ataattctgt actttaaaag ttttatttgt aatctttttt ttaatccttt 24000
atacaggttt gataacccag ctgctgtaag cccaactcct acaaggcaac taacttttaa 24060
ctacctcctt cctgtgaatg cttggttgtt attaaatcct tcagagctct gctgtgattg 24120
gaccatggga acaataccac ttatagagtc attactagat attcgaaatc tggccacatt 24180
tactttcttt tgttttctgg ggatgttggg agtattcagt atcagatact ctggtgattc 24240

ctccaagact gttttaatgg taagaaactt ttcttaactt ccaaatgatg ttaacattca 24300
gttgaataag taagaaacta cctttaattc agcaaaagca tctttttaga agcactcctt 24360
tctttgttca acggcatccc aatttataaa cgtatttgag tttatcccaa aattctcagt 24420
taatttgcag gcataatttg acatatgttc agcacataaa atatttgtta cctttatcca 24480
actagattat acactttttg aagaaattga ttatattgta tattatttgt tttcttggca 24540
cagagaagaa catcatataa gatatacaac tagaaatata gagcttttca gttctcatga 24600
gcaaggtggt ttttattact cccttagata gctttcacta tgttcggaga actctgatta 24660
tttgaacagt tttttttttt tttttttggt ggtggttttt ctgtactgtt tttcttctta 24720
ttttcttacc ctgtgtcact tcatactcat aagagttatg ttttctctcc cttttaatta 24780
ttgaagacag gctgggtgcg ttggttcttg cctgtaatcc cagcactttg agaggccaag 24840
gcaggtggat catttgaggc caggagtttg agaccagcct ggtcaacctg gtgaaactct 24900
cgtctctact aaaaatacaa aaattagctg ggtagctggg tgtggtagtg ggtgcctgta 24960
atcccagcca ctcaggaggc ggaggcacaa gaatcacttg aaccagggag gtggaatttg 25020
cagtgagctg agattgcgcc actgcactct agtctgggcg acagagcaat actctttctc 25080
aaaaaaacaa aaataaaaat aaaagtttat tgaagacaat agtacatttt ctcaggcatt 25140
tattatatgt cagaaagtat gtttaatgaa ttgcagatac ataatttttt gctgtagctg 25200
ctagattgat ttattcattc aataaaaact tagactttac cctttgtaca gtgcagcatt 25260
ttaggtgctg aggttatgat agtaaataca cagttcacgt ttccagagaa cttacacagt 25320
tcacgtttcc agagaactta cattctagag aaatttggat gtctgagata cagagtagat 25380
ttaaagattg tgttgtctat tatccttgaa catgttgagt ttgcattact gttgaaacat 25440
cctgttggac attttgaaca gcactttatg tctggagttc atagaagata gttaagttac 25500
acatagaaat cttatccatt aacatagagg tggtaattga aaccaggatg tagataaggt 25560
gaaatgtaga tgaggttata gagcagcctt tctcaactgg ggttttgtca attctgtcaa 25620
agatggtagc tgttaacatt ctagatgcac agaagttcac tcatactcat aatggaagcc 25680
ttagggtatt tggtcttaat tctccccaga aaacagttga gtgggctaaa tacagtgaaa 25740
agagaagata cctactacta ggtcttgaga aactcctata tttagatgtt gaagagaagg 25800
atcctgaata gaagacatat cgaatgaaga gaaaagtagg aaagtgtatt acagaccaaa 25860
tggttaacac taacagatgc tcttgagggg gaaagtgaga tctagaatct gttggattgg 25920
acttctttta gtctgtaggt acttaaatga aatttgttta tgtgcagtga gtgatttgat 25980
gaggaagtga agagagctag tatagacctg taggaataat ttaactttga ctgtttcaaa 26040
ggatgggata aacttgatta attcctccct aagggaagac tatgctggtc aggcaatggt 26100
ttcaagtata acaaactgct tatcttatgt agggcctggg gaagcattga gatctggcat 26160
tggtagacct tcagaggtag gagggagcaa tgtcagggag agactgttga ttggttggca 26220
tatagaagca tttttattgg cgtactcagc tactcagctg acttttttga atgcggatat 26280
ccctgatggg ttggcttgca ggaacatatt tattaagctg aaatgttgat tggttaagtg 26340
gtagacttaa gttgattagt taagtgggct acttgatact atggcaacaa aatcttttcc 26400
taagatcatg agaacttgtt ttgtacctga ggtatgtaac gaatagggaa ggtagcttaa 26460
aaaatgtatt gttaaagatg tttaaggctg gagagacaag agcatgttta aatatagata 26520
ggaagaatac agttgattag aaagtggtta ataaattaga gaagggataa ttgttagtga 26580
agtttatgag gagcaggaga tagggagcac aggtttggcc tgagatagaa gagacacccc 26640
ttatgttaca agaagaaatg aatgtggatg ttggtaggtt tagtagcagg aaattaaaga 26700
aattcttgtt ttatggcttc agctttcatt gtaaattagg aaatgtagtt gcctgctaaa 26760
actgacatgg gtcaaaaatt tgagaaaagt agtgaagatt caaagcagtc aagaagagtg 26820
gaaggaactt tccagagaaa cctagaaatc ccaaacagtc ttaaggataa atttgaactt 26880
ggtgctcatg cttttatgac agtattaatc tgttcagctt tgggactttc tctagcagtg 26940
cgttgccact ctggtaaata cagacaaaag aaataaacgg tagaatacat ctaaagctgg 27000
agttttccta aaagaaaggt taaaaaaaaa ggagaggtag gggaattaag gtttcagcaa 27060
ataaaataat ggtgtatgta atgtaagctg aattttaaat gtatataaaa atggaacagc 27120
tgtggcctta agcataactc tattttcagt atagtcctaa taaaggtgct atttttttta 27180
tggtgatgtt ttctgaagat ctctggaagg gataagtaga acaaaggagc acctaccaga 27240
taccatgtta agcacttatt agataagatg tcatatatat gtcaacagca taatgtaagt 27300
tgttattaat ccccatttta tttgtaaaga actaactggg ttcaatcgct catgcctgta 27360
atcccagtgc cttgggaggc agaaatggga agatcactgg aggccaggag tttgggacaa 27420
acctgggcaa gacagtgaga tcccacctct aaaaaaaatt tttaaattag ccaggcatgg 27480
tagtgcatac ctgtagtcct aggtgtttgg gaggctgagg caggaggatt gcttgagccc 27540
aagagttcaa gaatgcagta gactatgatc atgccactat actctatcct ggatgacaga 27600
caagaccctg tcagaaaaaa aaaaaaagga agaagaaaaa aagaaagaaa gactctggag 27660
gtgactgagt ggtttgccgt atgtaggaaa tgagaagtaa agcagatttg tctcgaactc 27720
tttgctcttt ctactaatat cctaacattt aaggacagtt tatgctactg gaagaaggaa 27780
attacaagag agtgatagtt aactaggttg gcatacatta ttagagagca tattaggacc 27840
tgtgaataag ttaatcatta gagacatttc ttatcccaga acctatagat ttttcctgag 27900

agattttttt tgttttgtat agtttatgca tcagtctaca atacactttt gagtgaattt 27960
aacacttaat tccctcaagt gaaattgcct tggggcggcc atgaattaat aggtataatt 28020
tttttttcaa tgaactacat acatattcag gaacttttct ttatctgttt cacatttttt 28080
gctgattatt tttgtcaagc atctctggga aatggaaagt gaattctatg cactcttagt 28140
tattcttacc tttttgattt aaaaaaactg aagtgtcaga cctataaatt ggtatgatgg 28200
tgaccagttt gatggtagtg attttcctca gattcagtgt tgccttatta caaatcatta 28260
atgtgtgaac atttgtggag agtattcttt tcaatgtata cacctctata tttcacaaat 28320
taagtcaaat agaaaaaaag gacattcttt caccttagcc cattagaaga gtctcaaact 28380
tgtgaagcta aaggtttaac ttgtttattc aattaagtac tgtaaaacaa atcggggtaa 28440
caaaatatgg aagcagatgt ccatagaaac agatttccta ataacgacca aacttatttc 28500
cttcacaagc tatttatgtg ctagtttttt atgggtgtat aatgtggaaa ccacatgtca 28560
tcccattttc aaaatggtaa gaaatctaaa catttttatg atgtttcatt tcaatgttca 28620
gtgttatatt aataatttta gaaattcttg tagaatgaaa gactgttgat ttaaaggggg 28680
gcagttttat ttttatgcat acactaagct tctagttaaa tacatttaac ctttcaggat 28740
gttatgtgaa gtaatgctaa ggtacagtag tctgctatct tgtctgaacg acacctgatt 28800
tcattccctc agtttccaat atcatgtggt ttcttaaagt agtaaagttg taaaaactga 28860
catgtttagc ttttttttaa tcagcatttg tggttttgtt ttgttgaaaa acctattagc 28920
atatttcatt atcatcttat ttttaccaca cgcacataag ttagatgtgt aaaattttta 28980
tttatttaat ttgtattcct cctaactgaa gttttatgcc ttttgaccaa cctctcccca 29040
accttctccc tccaaccctg gtgaccacca tgtactgtac ttctgtgagt tcaactttta 29100
caaattctac atacaagtga gatcatgcgt tgtttgtctt tctgtggctg gcttatttct 29160
cttaacatgg tgtcctccgg gtttatccat gtcagaaatg acaggatttc cttctgtttt 29220
aaggctaaat agtattctaa agtgtatatg taccacattt ttttcatcca gtaatctgtt 29280
gatggatact taggttgttc ccatatcttg gctattgtaa attgaataat gctgcaatga 29340
acaggggagg tcagatctct cttcaacata ctgacttcat ttcctttgga tctataccca 29400
gtagtgagga tgctgggtca catggtagtt ctatttttaa ttttttgaga agccttccta 29460
ctgtttgctt aatggcaatg ctaatttaca tcccaccaac agtgtgcaag tgtttccttt 29520
acgttcttgc caacatttgt tatcttttga ctttttgata atagccattc ttacaggtgt 29580
gcgatgacat ctcattgtgg ttttaatttt cacttcctta atgatagtga tgttgaacat 29640
ttatatactt gatggccatt ggcatgttgt cttctgagga atatctattt agctactttg 29700
cccatttttt aaattgggtt gttttcttat gattgtttga gtgtgttatt ttagatagaa 29760
tttcaaaaag tacttccact atttaactgg atgaaccttt attgtttaat ttttgcttaa 29820
agatgtgaat atttacattt attaaagttt ttatatttta ctatgcaagc atattcttta 29880
cagaaaacca gtgagatata gctaattatg cttctcaaat ttaaaatgct gtaatggttt 29940
agattttaat ttcaaggagt aatcactccc aaacacacct agaaatcttg ttttcaaagt 30000
tcatagatat cctctaattt taacttttag aattcaagat ttccatttca ccttgcttac 30060
gtacatcctg atgtttgtta atgtctaagt tgccttcttt atgtttaaat taatattctt 30120
cacagtatta ttaaaaatag tttagcagta cactatccag gttaaatgat aaaatatttg 30180
gattcaagta tcagatgccc atatatttct taatttatta taaattattt taaatttatt 30240
ataaattatt aaatcttctt tttttttttg taggcgcttt gtttaatggc attaccattt 30300
attcctgcat cgaacctttt ttttccagtt ggatttgttg ttgccgagcg agtattatat 30360
gttcccagca tggggttctg tattttggta gcccatggat ggcagaaaat atcaacaaaa 30420
aggtgaattt aaatgtcagt tcatgtaatg cttactatgg aattaagtgt gacacatttt 30480
aaaatttaaa tatttagttc agttttctaa gtcatgcttt tgtatctttt ggtttttcat 30540
ctccatagtc tcctcatttg tcttgtaagt caagttagga aagctccatg gcacctactt 30600
attctgtgtt cttccctata tttatgtaat ttgattttac agcctgagac agaacaaact 30660
ttgagtcaga tccaaatgaa aacaaggatg ttactgagaa taaagtctca aatgaccatt 30720
gccacttcca tattcagtga actaagataa tagtcatcct acttaagtga gtcatattga 30780
gttaatatat ataaaatgca tatagtaagc aatctgtaat ttcttgctat catcactatc 30840
ttcattataa tctttgtatc ctagttgcct caaactaaca gttaaggaac agcatagtca 30900
cactgtatat atgcttagga actataattg ttataggtat tatctcattg tatacttatg 30960
ctgtggcact aaaattatca aatcaagcaa attgttaaag gtcaagtaat actaatgtaa 31020
taatattgag tttgcaactt gaaaaagaaa tagtcacttc tttcactttc ctattttcca 31080
gtcatccctc accccacaga attctgactt ctctccactt cacccttaag catataacaa 31140
agtgcctcct agtagctaaa ctaagtcaac tcttttaatt taaagtttat ttccctagtg 31200
tctgtaaaaa gtatttgtcc tgattaattc ttctggctgc acctgctctt tgaatattta 31260
catttttcaa agtcttatgg ccactttttt cacaccgcat gtgttccaca gataatctca 31320
tccctgtctg caatatttaa ctgccatcta ccaaacctgt tctcttagct tttacccata 31380
tatccattta taatcactac ccatttttga atgcctgcca tgtaccaagg gccatgatag 31440
acactttaga tttcttttct catttaatct tgacacttct taagtctgct ttgcagatgt 31500
gaacattgaa gccatatagg ttatccaaga tcacacagct agtaaataac agtcttgttt 31560

cagattcaca tctgactgct tccaatgacc atgttctttc acatccatac ttgtgtgttc 31620
tagttaagtg gatctatcaa aatgaaataa attaaaccat gttacaggaa caaaaacaca 31680
gactcagaga ctattaatac taaacaagat cataaagatt ataggtgaag aattgagccc 31740
tcaaataatt aacctaaagt tgtataagta atttgcctct gtttaatgaa gagtcacttt 31800
tactatgcct gttaatagac aattctagct ttacaataga gaaataaacc aaaaatatca 31860
tgatagtctt ggtataaatt gtaagtaaaa cattttcaaa tgtgatcagt gataaggttg 31920
acccctttct aaactcaatt aagaatactc agttgtagaa aatggcacaa aggactgtaa 31980
gtgtaaaaag tcacgtttaa aaattggttt tttccatttt ttatcattta ttttaaacag 32040
agcttttgaa atatagccaa tattagcata tataatgtaa ttgactggaa ggtcatattc 32100
acagtattta atttttttgg tgttgtatcc tctaaagttg ggtgttcatt agaatgaatt 32160
agaataaatt aacatatgat attttaggag catttaagat acttcttacc tctgttctaa 32220
aaggtaaaat gcttttggtg atgaagacaa ttttttttgt tttgcttttt ttctcttaaa 32280
cagtgtattt aaaaagctat cctggatttg tctgtctatg gtgatactca ctcattcctt 32340
aaaaacattc cacagaaatt gggattggga gtctgaatat acattgttta tgtcagcctt 32400
gaaggtaaag tgttgttcag aatgacagga aagtatgtca tcagtgattt cttggaacaa 32460
tttctaagct gttttaactt tgtttttaat tagacattta acagtcaata ttaagtactt 32520
tactaaaaga ttaatgaaaa taatttgttg tttaacataa tgccataacc aaatgctctg 32580
aataatactt agctatatgt ccaaagcata tatgataatt ccctaaatgc tatacagtta 32640
ttggaagctc aaatgacaca atgtttaaat cagaatacta tatttcattg gttctaaagg 32700
tgcttatatt tttccttttt tatatctctg aaatcaaata agtcttacag tgaaaagcat 32760
cttaaattag tggccttttt tctctcttaa tggtacttaa aataatgatg ttataatcga 32820
tggcatcaat tcagtaaaat aaaatgtaca gcgaaaaaaa aaaggcaccc aatgatgtgg 32880
aagataacca gtggttacaa tactatcaga aagataagaa ttggcagaaa gaaaggatca 32940
agatttctat agaatggaca ttataaatcg gaaaaaggtc ttttaaaaaa caaaaatttg 33000
ttttctttaa aggttatccg caaagatttt ttcaagctct cccttaccag tacccctctt 33060
cagatatctg aatttttact tcctttggac tgtttcttaa ctcttcatta cttagcttta 33120
ttggattgct gcttcattcc agtggtctgt gtgatttctt ggttccccat ctccctaaga 33180
tcagatgagt ctcttcttat acaactcagt agctctactc tctctcccaa ctcagaagtc 33240
tgccaacaag tgagacctca ttaccatgct tattaatcca tactcctagg ataccagtgg 33300
agccccataa ttttccagaa ttttgcttat actcactccc aggacagtgg ttggaaccta 33360
tagccacaac gactgcaatt agttccttgg ctaggtgtca tctatctgca agaacactac 33420
ggcaatctgc gaactctgaa agcacagatt gactcagagc tttaatctca ggttttccat 33480
taccataatg gactacataa ataattttaa atatgtcaag tctaaaaata agtcatcagt 33540
gcaacattta aacatattct catgttccag tgcttttgct cctaaaaata caaatggcag 33600
agcagaagtt ttgaaacacc actgtgcttt catcaaataa cattttcagt aaaatgttaa 33660
tatataacgt gaatgaaata agacactgct gtggttttag tatgttggtg ttcaggttca 33720
gggagagtaa atttggtgaa gcagtcttta tttgactcct gtgtcatccc cagagacatt 33780
tccatagaac attattttct taatagtata ccaaacaaat atctgtatga actggagtca 33840
ttttttttta attgtaactt tttctatctg tgcttgtatt taaggtaaat aaaaataatg 33900
ccaaactttg gaataatgtg ggtcatgctc tggaaaatga aaagaacttt gagagagctt 33960
tgaaatactt cttacaggct acccatgttc agccaggtaa gcattattaa ctaataaaat 34020
catgaatttt attttttaaa aagttttgat tatccttggg ggaaataagg tagtttattt 34080
actagcttta ttctgagtga cttgaatact agagttcagt gcttattttg aactggatta 34140
catataacag ccacttaata tcagactctg ttaccaggct ggtggctcac acctgtattc 34200
ccagcacttt gggaggctga cacaggaggg tcacaccagg agttcatgac cagcctaggc 34260
aacaaagcaa gatcctgtct ttacaaaata aaaattaaaa aattagccag gcatggtggt 34320
gtgggcatgt aaactcggct acttgggagg ctcaggtggg aggattcctt gagcccagga 34380
gttcaaggct gcagtgagct atgatcacat gattatacca ctgtactcca gcctgggtga 34440
cagagtgaga ctgtgtctca aaaacaaaca aaaacattta tggatttttt cttaacatag 34500
agttgttgtg tggcatggat atggataagc agttttggtg gaatgatggg tgcaaaagcc 34560
tgatagtagt gagcttaaga gaatggggaa agaatacttg gagacaattc gtatagaaaa 34620
ctcttttgaa gggtgttgat aagaaatggt ggcagtgctg ggtatgtggt caagaaagga 34680
tggtaagata agagggtttt ggctttttta aaccagtttg ggccagacac agtagctcaa 34740
gtctgtaatc ccagcacttt gggaggccaa ggtgggcaga tcacttgagg tcaggagttt 34800
gagaccagcc tgggcaacat ggtgaaaccc catctctact aaaaatacaa aaattagcca 34860
ggtattgtgg tgggcgcctg taattccagc tacttaggtg gctgaggcac aagaatcgct 34920
ggaacctggg aggcagaggt tgcagtgagc caagatcgca ccactgcact ccagcctggg 34980
tgacagagtg atatactctg tctcaaaaaa aaaaaatagt tacatgtttt tctgttgcaa 35040
ctttttagct ataatagtga tggcatagac atggaaaatt ctgttaaata ggattaaagt 35100
tttttcagtt gtgtattact catcaagaaa gggtttaagg agtgaataaa tgatgagcca 35160
cagaatctaa gctggttaag aagggatgtg agagaggatg agggatggag gaaaggtaac 35220

aagatcagtg gatctgagga attgttggtt tctgagtagg atgtagctag agcagcagta 35280
tttttcgaca ggagtgctat tggcgttttg agtgcagaac tgtattacag gatatgtact 35340
agacatggcc ccccatccac caaatgccat cattattttc cattcagagt gatgaccaaa 35400
aagaaacccc tgcacattac taaatatcct tgaaggggtg cagagtgtca gacactgttg 35460
tttttttcta gtaagtacaa ctatgaccac tgcactaact ggctaaggta gaaaggaggt 35520
gaaaatcatt tggaaaaaag gtcatcaaga ccacagtatt caaagattaa tatctatatg 35580
gattatgaaa tcactaaatc attaagaatt ttgacaaaag taatgttaga gaaagcaaca 35640
gggagccagg gtctgtgatc caggaatcaa agactacaac actgataaga atagcagata 35700
atagagttaa attacaatca gacatgagta tggtaaaaag gagagagaat tgtccaaaaa 35760
tggcaacatg gatcaaggaa gaagatacct gttcttcccc caggcccagt gtggcaaagg 35820
atagaaaaga acacagtcac cacttgaaag ggctaaaggt aaccatgtct ccaaaacaaa 35880
gctaagtttc aattagagaa aggtgaagaa aaagttgtga aaatataact gatggtctag 35940
cctttcagtt ttatctctct tagcccttca tgtagtttag tttttcttgg aatgtgggat 36000
taatgtcctg tacttcctta cttctttgcc tttattttct tccttgagac tagaatttct 36060
cccttctccc tttcctccca ttttccagtg acttctcttg taaaaactct tcctatcttt 36120
ccagctgtaa gcaaatagtt ttcatatgta aaacccctta acttcctctg tatctttcct 36180
atatttgtaa ttttctattt agtgctatat tttatttatt taaatacaaa tttatttaaa 36240
atatttattt atttaaactg tggagcttcc ttggggtagc aggaacatct gattcatttt 36300
tgtacccctg catcatcaag tagggtccta tacgcagtca gcagtctcta aatattttaa 36360
ttaattactt tcttaagtct tgagatgtgc aatgcatgga ttaattatat tctaagagtt 36420
tttagagaag cagccatctt tgattgtcag gcacacaagg gctttgagac tgagatgtta 36480
cttaggcccc tcagactctt ctttcttgtt tctaaaatgg gatagtaatt tctgtctcac 36540
agtattgttg taaatactaa gttaagtgtg tgtggggtgt gtgtacacat tcagttttgt 36600
ctagtgaata gtgtgcctat cacaggatgt tgcagacatg gacactcagt ttttaaataa 36660
tttttccccc tcattgtcaa ttgtcactaa ttgctgtttt tgagatattt gctgaacctt 36720
tcttcagttt gacttcacta tcacactttg agaattgctg tttctattaa tcataagcca 36780
tgagatagga aaacaagtaa accagtttat tatattatga ataaagcgtt ttttaaactt 36840
agaaatattg aatgtggcat ctgaggtttc ttggcttatt ccatgttttt ttaagctgta 36900
ttcttcctag atgggtttga cagatgaaat gacatgttat tttactgtat cttagatagt 36960
ttagacgggg caagacagtg accaggataa ggttaagagg gacaactagt gtgatggtta 37020
gttaaaagtt tggactctgg catcagacct tttctcaaat ttagttcaat cactctaagt 37080
tttctatcta taaatttgga gtaataatac ctccattctt ttgtatgggt tatgttaaat 37140
actggttata taagacttag gccagtttct aggcaataat gtatattcaa taaatatgag 37200
ctggtaagta ttaatggcat tattttcact ggagccagga aaggaggttg ctgagtctgt 37260
cctgcagact ctggctgagt gatggatgaa agaggtatgc tgacacaggt attttgcctg 37320
acagtgtggc taggagactg cacagcttag caccgccaat gagagagtgc agcagccacc 37380
aagagagtgc agcccccgta agcaggcccc ggtcgcattt atttaataca gatttaatga 37440
cacaggcttg gagcaaacac aatttgtggg taataaacat tgtcaacccc cggagtagag 37500
agcagtcctg tgcgcaaatg atcaaagatt ggtttctgga gacaggagta aacaaattta 37560
tctagataag ttcctttaca ttcccttgtt atctaccctt tgctctcagg ctctggataa 37620
gagaatctgg ctgccttcag ccataattct cttctgaagc ttttgcaaaa cctcctggcc 37680
ttccaaaaag atttgcatct ttccctataa ctttttctta caacttttcc cactaccctg 37740
accgaactcc tacatctcac ccttttctgt tttttgcatc aggttttgtt gattgaagag 37800
tacagatgtg tgcagcaaca ggtttgtcag gcacagcagt taacactcgt attccggctt 37860
tgcatcctag aattagtaaa taacataaga caaacatgag tataatcagt aatattcttt 37920
tccaattaag gagtgacatg tagtgttact tggcacctca gtccagtgtg tgtcattact 37980
aaggaacccc actgggggta tgttaatccc tcctagccaa gcagttacgt tattagaggc 38040
cgggaagggg gtgtctgccc aggtaacagg gtgaaagaaa ggcggattta gaagatgggc 38100
ctaatagaga gtagcaggta cagatagcag gaaaagtgag agaataagaa aaaccaatgt 38160
cctatgagag ttgtaatgta caacagaaag catagtaagg aacaaattat ctggagtgaa 38220
tggtgtctgt gtccagaaca ggattccctc agcctcctga gttatcttct tcagcatccc 38280
gcaggtaatg tttggggctt gtgtcattca agaaaaccgc atcttctggg gctgcaggtg 38340
ctgcagggtc gtttccttca tttctggtac caggttgggt cctagcgaca ccatagtatg 38400
gtttgatgca tcatgctgga atccatagag gacttgaggg ggtgtgaaca caagcatatt 38460
ctcttcccca tgtcaacaaa tcatttggac cacaccatac attactattt acatctttcc 38520
ataaaaccac aggttttatg ttttgaagaa ctttagtgaa atgcttttct atggctgatt 38580
gaaatttatc atctaaattt taaaaattaa gggtaaataa ggcttgtgcc aatagtgttg 38640
caggatcctt actcatattc ccccttttta gtttcttgag catattttta agggtagagt 38700
ggacacgttc tactatggcc tgtccttgag gttataaggg atacctgtgg aatgttggat 38760
gttccacgtg tgacaaaatt gttgacattg tgagctggca taagccagac cattatcagt 38820
tttaattttt gtgggccacc ccataaacac aaaagttaag agaaaatgtt taataacata 38880

ttgggaggac tcttcaggaa gagcatgtgt gctaattagg tgagaattgg tatcaatgga 38940
tacatgtata tctaagtttt ccaaattcaa ggatgtgaat aacatttgtt tgccataacc 39000
gattaggttc tagtcctcta gggttaacac ctgttgaagg aggagacatg cctgtgagtt 39060
ggcaatgtgg gcattgtaaa ataatttgtt tagctagtct ttgggtaggt tgaaattgtt 39120
tagttaagtt tcttcagttt tggtggaaaa attgatgcaa ttgggtggtt tggtcaagca 39180
gtgacgtcat aacttgcagg tctgcttgtt cattgccata agccagtggg ccaggcagtg 39240
agctgtgggc tcgagtatgt gtgttaaaaa taggatatgt acattgatcc agcaattgct 39300
gaagttgaag aaaaagtgta cacaggtggg ctcgagagtg gacttaatga gggctgtctt 39360
aagcttctgt aataaataga gtaagcagag tcattaacaa tattgataag ctgagcagaa 39420
aaggtctcca gggccaatat taaggctcca acctcagctc tctgagtgct ggtaaatcca 39480
gaacaagtga gggaattatg cagtttccac caaatagcca cttttccatt tttaccagag 39540
ccatcagtga aaagcgttaa agcattgggt atgggggagt gaactacttg tgtagacaca 39600
actacaggag tacgagaaaa gaactgaagt agtttgtcag caggaagggc atgctctgca 39660
tggcctgcat aattagagag tgctatctga agatctagag ataggggcaa tattgcttcg 39720
agttgctttt tactcaaagg aattcttatg acatcagggt cataacctag aaactgattg 39780
catcatctgc agcctgtata ggtgacttta ctaactagct ggatataggg agatagtgtt 39840
ttagtcccag tacgtgagca aaaaacccat cctaggaagc atagccctgg ggctgtctgt 39900
cctattaatc ctgttgggga atgtttagta gggaaaacaa acaattgaac tgaatattgc 39960
aggtctgtgc aatctagtta cctctgagaa acagcttgct ctattttttc aatttccctt 40020
tttgctgcag gagttaaata cctgagagag tctaaggctg tattgccctt taggatagaa 40080
aacaggttat gtaacttatc agtagttatg cccaagatgg ggcaaagcca attaatatcg 40140
cccagtaatt tttgataatc atttaagata tgcaagttga tagtatttaa tttaaccttt 40200
tgaggtctaa ctgaccagga agttagtatg tatccaagat attttcaagg agaagacaat 40260
tgtacttttt caggtgctat gactaaacct cttagctatg tattctttac gacagaggct 40320
tataaactta aaagtactgg ctccgttggg gatgctagta aaatatcatc tataaaatga 40380
ataatcttgt atttaggaaa ttcttttcta cttgggagca aagcctgatt tacatgatac 40440
tgacacatgg taggactgtt cagcatccct tgaggaagca cttcccaatg aaatcagtga 40500
gctggccttt cattattgat agctggtatt gtaaacacaa atttttctct gtcctgttct 40560
gcaaggggaa tagtacaaaa acagtccttt aagttcataa tgactacagg ccaatcttga 40620
ggaatcgcca cgggggaagg gagaccctgt tgaaggggcc ctataggtta caaattagca 40680
ctgatagccc gtaagtcatg cagaaatctc catttgccag acttttggga aatgatgaaa 40740
atgggcgaat tccaaggcct gtttgacggt tctgtatggc tggcttttaa ttgctcctca 40800
actaattcat gggccaacta attcatgggc tctttgtaat ttttctccct ttaaaggcta 40860
ctgttctacc caaataggat tttgagagag ccatgtcagg gggaggagag gaataacagt 40920
ggccattatt agaaagaggt ctgcagaatg acctcagtta accctctcat aaatgggcta 40980
gcagctgttt tctttaatgc ttttccttat ctctttataa gtgccgaaag taatgagttc 41040
atatacccca ttgccttgtt gatcttgcat tgccaggcag gctaagagct ccccttctaa 41100
tgctgctacc taagacaggg tcccatagct atagtgtatc ccttgtcttt cttccaattt 41160
attggaggag gggcctcagg caaaacctct gtttcccctt tggtattttt gccctttact 41220
ggtggggctg agggagaagg aggaggcggt aaggtaggtg acggtttctc ctcccttccc 41280
tttttaggtt attctgtgta gtgcgggacc aaagcagccc taactaaggg ccataacatt 41340
agagatgcta ctgggacctg atatccttgg gcatgatgtt aagatttctc cacacttgtt 41400
ctcagagccc tatgtctagc ataccttctt ctgggaacca tgggttatgg gaaacagcag 41460
tttgcattat gtcccttaat tgagcctgcg aaactggggc tccactagct ttaagcagct 41520
gtttcagtac ttttatatac tgtttctgtt gagctgacaa ccactgtccc atgatgaaac 41580
cctagcctga acaattccct cgaacttgga aatcccaagc gggcaccaat gacttactgt 41640
gcagtctctt aaccttcgtt tttgagggtt ccatcgcaat ccattgcagc cttcctcaca 41700
cggggcacca cctgccgagt ctgtcccaca gactctggcc gagtgatgga tgaaagaagt 41760
acactgacac aggaattttg cgtgacagcg tggctagggg accgcatggc tcagcaccac 41820
caacaagaga gtgcagcagc caccaagaga gtgcagcccc cctaagccag ccctgctagc 41880
atttatttag ttcagattta atgataaagg cttggagcaa acacaatttg tgggtaatga 41940
acattgtcga ccccccaagt agagagcagt cctgtgcatg aataatcaaa ggttgatttc 42000
tagagaaagg agtaaacaaa tttatctaga taaattcctt tacattccct tatctactct 42060
tttctctcag actcctgata agagaatctg gcggccttca gccataattc ccttcggaag 42120
cttttgcaaa gcctcctggc cttccaagaa ggtttgcatc tttccctata actttttctt 42180
acaacttttc ccaccaccct gaccaaactc ctacaggagg tggcaaaaaa gtgatgtata 42240
atgcagggaa gaaggctgaa agagaatgga tagcaaatgg ctctgtaggt atatttgttc 42300
attgggcacg tatttgagaa tttaccatgt gtcaagacac tgttttaagt gctggggaat 42360
ccagaagaaa taaaacaaat ttcctccctt cgtggagttt ccattctaaa taaaggcata 42420
aacataagta aacaaataca caccacatgt aaatatatat atatatacac acacacaaaa 42480
tatatatata cacacacaca tatgcatgca catgtgtatt tatatgaaag tgataaaagc 42540

tataaaaagg gcatagagat tagaaatttt cagagaaagt ctcttcgaga tgataataaa 42600
cagagcaacc ttcgcacata tactctggtg taattttaag tatcactggc tatgcagaaa 42660
acagtgaaga aaagcaggaa gatcatttag aaggcttttg cagtaaccca aagacatgat 42720
tgagttgtaa ttttttaata gcaatcttga agtacatgtg acatacaata gactatacat 42780
gtttaaaata cataatttga taagtttcgg catatgtata ttatctgtga gaccataatt 42840
acaattaaga tactgaaaat atccatcaac ccctcaaatt ccttggatcc ttttctgata 42900
cctccctctt accccttttc accccagcct tctctaagca aaagctttct atcactacag 42960
ataatgtgct ttttctaaaa ttgtgtatga atggaattat actctatgga ctctctcttt 43020
tttttttttc ttggcttttt tactctgcac agttattttg atactcattc atgttgttgt 43080
atgaatcaaa aacccatacc ttggtatggc tgagtagtat tccttcacgt agatatacca 43140
cagtttgttt atccatttac ctgttgatgg atgtttggat tgttcacagt ttggagctgt 43200
tacaaacatg gctgttacga acattcatat accagtcttt atggatatat gttttcattt 43260
ctcttaggta aaaacctgag agaaaaatgg catggtcata tgatttctgt atgtttaact 43320
ttttaaaggc tgctaaatta tgttccaaaa aatgtatttc ccaccagcag tataggagag 43380
ctctcctgcc tttcttaatt cttcatttgc tgagaatttt taacacaagt aaatgttgga 43440
ttctgtcaaa tgcttttgtg caccatttga gatgatgata aggtttttat tttttttgtc 43500
tgttaataca gtggatatca tcaattgatt tcataatgtt aaaacaacct tgccttcagt 43560
aatacacaat ttgattgtga cttattatct ttattatatt ttatttgtta aaatgctatt 43620
agtaattttt acatctatat ttatgaagga tagagagttc ctaggagatt ccagatattt 43680
ataaatttat ttttttaaat aaattgataa ttttgtggac aggaaatact gaaactctac 43740
ataattgcct aagccatcaa tgtgagaata cttcactgtt gggcagctaa tatttcactg 43800
ttgggcagct gtgtattagt catacacagt tgcctgaaaa cttcccagcc acatcacatt 43860
tgcatcccta ggcaggatga ctgagatcat atgtgaacca gtgtctaata actactgtca 43920
tttcaaataa atggtgactg taaacctgtg tacacaggtg tttgttacta ttaaaaacac 43980
atgcaaacat tcatgcaggt aagttgataa cctttagcag tgaggtaagt tcccttctaa 44040
acaaagttcc tttgttttcc tagtgaaaat atgatattag agatgtgagg agagataaga 44100
aatgaaagag actcaactag gattctggaa aaattaactg attatagaaa cctagtattg 44160
acaaactgag actacatttg agatttgtga ggtcctcatt taccattgaa ttaaaagcta 44220
gattgttaac tcatgctgct gttgttttgg ataggtactg gtaagagtgg gcttgttctt 44280
tgaagttcct ttagtttctt tagtcattta aatgtcattg attttttaca tcttgccttc 44340
ttttcttacc ttttgttaat acttaaattt atcttaaaag tgatactatt ttaccctcta 44400
gaatcttaaa actttgtaaa ttataaagaa ttgcttagaa attaggtctt tcattttatt 44460
agctgtattg agtggccttt ttgatgctaa acaatatcta attgcatctc tgctctttga 44520
aaacataggg ctgctatact ctagtgtctg aagtctacat taatgacaat atctcctggc 44580
cttaattcta agagcatgat ttcaatggat agtaattcac ctcatttttt atctaagtat 44640
ataacctctt tcttttctaa cattggttgg ggataatttc aataacattt taaatactca 44700
gctggcataa ttacagtttt attttaacaa attttcagtt tattcttaaa ttcttagtgt 44760
ataatttttt caggttctat gtaccttagc ttttcctaaa taacattctg agctctataa 44820
acatatggta tggatttatc tggtaaaaaa aaaaaaaaag atataaaagc aaagactacc 44880
ccatattttt aattgacaat aatagtacat attcatggga tatatagtga tgttttgata 44940
catataatgc aagcaaatac cctgatctaa tcactatact gcatatttta tatgcaattg 45000
gataatacaa tgattttcag ccctgattct acatcagaat tgcctttgac actttttatg 45060
aacatatttt tgcagatttc atttctgaac attatgtttt gatggaccta ggtggggctg 45120
aagtatctga ctttttttta aactacacag gtattctgat ttgcagccag aattaagaat 45180
ggctagctta aacgtttttt ttgcttgacc cctcctggct ccatgagact cagcaataca 45240
tctaaaaaga aaaaaatcta cttccttact cctaaactta tctataatca aacctggacc 45300
gcatctgcca gtttttgtgc agtgcacaca attgccagcc taaaacatta tatagcaagt 45360
tcttaggact gttactcagg gaatgcccaa gatatatatg cttggcccct atgtgtccta 45420
ggagttgcag gcaaagaatt attgcacaat tgcaaaataa ttggtatcac aaggcaagta 45480
gtaatcataa tgaacctttt catgtatttt agttcgtgtt gagtataaac tgtctgattt 45540
aagatcttct accttccttc tatcattatt ccaaaccttt gaaatattta ctggcaagta 45600
tatcttttta aaaagctact gtacaatgaa tcctcatctc tttcctattt cagctacatt 45660
ttttttccta tccctgaata aatttgctgg gatttttact ttccacatca gaaaatgaga 45720
cataagttcc tgacattcta aaattaacac aaccttttaa aaaaatctaa aattaatttt 45780
aaggagttaa ttatcattta aaagagctgg gccactgcat cttaagtaat actattatca 45840
ttatgttcca ttatactctc attctagcca cagtcacaaa aatttttaaa aatcgaatgg 45900
caaagttttt tttcagtctc tttgcccctg ttccaccatg atgcatattt gttataacac 45960
ttgaaataat tctaaaaatc ctgatgattc attttacttg tagagttgag ctgtaccttt 46020
tgagtgaatt cctaagctat gagattaagt aaatggaagc aagctttagt gtagtaaaat 46080
acaatgaagg aaggtaataa aaatttcttc attaccttta tctctggaag caagctttag 46140
tgtagtaaaa tacaatgaag gaaggtaata aaaatttctt cattaccttt atctctctta 46200

tgtctcaact cttcaaaaat ccattcaaat agttttttca tgccatgtat gtattttctg 46260
tctttacatg ctttaagtaa ttttgtgtat accattggtt ttaagttata acaagttaaa 46320
ttacaagaac tttaaggaag aagcaggacc tgttgcctgc ttttgcattt ttgcctagtg 46380
tgtttgtttc tggaaatatg tcttctcatt agttacattg aatacttgtt tatatatcaa 46440
atacatttaa tcatttcatt gagtatattg aatatatcag ttgtatactt gccaacaaat 46500
gattgtttta aaattttaga tgatattggt gcccatatga atgtaggaag aacttataaa 46560
aatttaaata gaaccaaaga agctgaagaa tcttacatga tggctaaatc actgatgcct 46620
caagtaagtt gccataattt atctattgtg tcatatctat tagatgtcca tattgaatag 46680
aattatctag aaggaaaatc tttatctttt cttcagttag ttgatataaa agtaagtttt 46740
ataaagaatt acatttttct agcttactta attttttttt tttttttgga gacggagtct 46800
tgctgtattg cccagactgg agtgcagtgg cacgatctca gctcactgca acctctgcct 46860
cccaggttta agtgattctt ctgcctcagc ctcccaagta gctgggacta caggtgcctg 46920
ccaccacacc cggctaattt ttgtattttt agtagagaca gggtttcacc atattggcca 46980
ggctggtctc gaactccaga cctcgtgatc tgcccacctc agcctcccaa agtgctggga 47040
ttacaggcat gagccactgc gcccggccgt agctcactta attttaaaaa ttgtttttat 47100
agtccatttg taaaaactaa attcataaag tatgaatttt agatattctt gattatgagt 47160
tatcagcaag ttaatgactt taaataaata taattttatt tatgtatatt aagcatacag 47220
ttcaacatta ttcctatcaa aaaataaagt gataaaatta gtagatacat ctcttactat 47280
attgtgtcca tcagttgtta gaataagatt cattaaatat aaggtagtat cgaaattgga 47340
agatcttacc ctatgtcaat tttcatgtta tcagcaaaat atttctgggt tgtgaccgta 47400
ggatagttaa tttattccag aatactaagg actcacttgt ctggtgaagt ttggaaatta 47460
aacagtctga ttattaagtt aaggttttta taatacatag tatatatgga tcgagacttc 47520
cagtgaacac aataaattag atatgataaa aaccaataac aaatacaatt taactgttac 47580
agttaaggat taaaataatc cttaaaatgt catacagttc aactttaaaa aaaattaact 47640
atcctatcac tattaatatt actataaata ttattacaat tgagaaaact tcagcccagc 47700
ttaagctatt ttttctgagg acataagact atgccactaa ttttaccact ttgttaaaaa 47760
taaataataa aaatgtaaaa gaaaacacac acaaaaatgt gaataaaaat atttacttta 47820
cagtatcctc aaaaatgtaa ataaaatagt actttgtggc attaaggata tattttttca 47880
ttcatgccac ctcagtggat tattagcttt ttatttgtat ctgtcttttt ctgaagtcaa 47940
acaaatcaga acttttagaa gtcaaggtat cttaggttta gtcaaaacag gagtagaacc 48000
ctgatctctc agtccagtgt attttttaca gtatgttctg agttattttg aattagccaa 48060
taggaatatg tacttttgat ttttgatact gaaatatcaa atcatgaaaa tgccttttct 48120
tctaaacaga ttattcctgg taaaaaatat gcagccagaa ttgcccctaa ccacctaaat 48180
gtttatatca atctggctaa cctgatccga gcaaatgagt cccgactgga agaagcagat 48240
cagctgtacc gtcaagcaat aagcatgagg cccgacttca agcaggctta cattagcagg 48300
tatcccagtt caactttaag ctatcattat ggaatattga aactgcatct ccatgtacat 48360
tttgaatgaa ttggtttttt ttgaaaaaaa attatgtttt taataatctt acttagcaac 48420
caagtgcata gaagagcata actttttaga ctgacagtca gattttattg atttgaaatt 48480
aacaagggga atccctaact tgtaatagcc aacttaggtt ttgtattaag gaaagggtag 48540
aagcagctct gtcttagttg cattttcttc ccatcaccaa aattgttctt ttcctattta 48600
aattaaaact tgaaacttac attttataat agttacttct aatatcaaac tctcaggaac 48660
tttcttgttt ttagagttga ttggttattg atgcctttga cacaaagaaa acatcttggt 48720
tctcagcaaa aaaaaaagtt ttctggcacc acatgttaaa acttcattta ttttctcatg 48780
tatgtgttat catttgtatt acttttgagg aaaaatgtgt ttttaaactt cgttttgtag 48840
cctggtccag aaaactaata gtaatttgta acattttttc ctttggggaa agtacattct 48900
gaatttaagt ttttgagata aaatctgttt gtaagttggg gttttctttg ttccgccgtg 48960
cctattgagt ttattagcag ggttgtatct ataaataaca cctgtcatgt attaattcat 49020
aaagaactat tcaacaatct ctacattaaa aaaataatat tttggacaaa cattcattct 49080
agaagtggtt ttaatgagaa tattaattgt tgttcctttt atttctgtgc tgatactata 49140
ggaaagagta gtattataag aacaactctc atatcactac ttaaatatta tttgtacata 49200
tgtaaaaata attctttaag caaaattact ttttaaaatt ttctaacaga aaatagaacc 49260
acactagaaa gatttataca atggtttctt aactggtcct caattctaag tgtgttcctc 49320
aaagttatct acaggtcaga ttaatccctc ccactctacc tgttcagctt ttttttagtc 49380
ctttttctgt ttgtttgttt gtttgtgaca gagtctcaca ttgttaccca ggctggagtg 49440
cagtggtgtg atctcggcta actgctacct ctgcctcctg ggttcaagta attcttgttc 49500
ctcagcctca ctattagctg ggactacagg cgcgtgccac catgcctggc aaatttctgt 49560
atttttagta gagacagagt tttgccatgt tggtcagctg gtctccaact cctgacctca 49620
agtgatctgc ccacctcagc ctcccaaagt gctgggatta caggaacgag ccaccatgcc 49680
cagcattttt cttttttttt ttttcttttt tttttttaag aatggtcgta ttataagaag 49740
acattgatga ggactaaatt aatataagaa ggggttacag atttttaata ttttggtttt 49800
ttgcatagtg agccttctat tatacattca aatattggaa tttctcttac cactactata 49860

tgtcattttt ttcttctaat gctgttattt tgtcacctga caaattcatt aatctttcac 49920
tctggctagc tgcccttttt ctgatgcagt ttagtctgtc tcctttttaa gataaagtca 49980
aagaatatat ttgctctgtc ctccttcctg gattcctcct gataggttgc cctctgaaat 50040
caggcataat ttttttcttc ttaaataata tttgaaaata tatcaagcca aatatactga 50100
actaaggagt tcagatttga ggacagtttc aaagagttgt tttcgaaaga gagtttttta 50160
agagtctgag ttaagagaaa aaaaagctgt atgtggaaag gcttaaaact tggcaaaaat 50220
tgtatctaca aactgagatg gtaatgttgt aagtaaagct tgtgtttgat ttttccacag 50280
aggagaattg cttttaaaaa tgaataaacc tcttaaagca aaggaagcat atcttaaagc 50340
actagagctg gacagaaata atgcagatct ttggtacaac ttggcaattg tacatattga 50400
acttaaagaa ccaaatgaag ccctaaaaaa ctttaatcgt gctctggaac taaatccaaa 50460
gcataaacta gcattattca actctgctat agtaatgcaa gaatcaggta tgttttctca 50520
aaatatttct gtttatataa attgtagttt ataatataat aagaccttaa tttaacttta 50580
ttacccatag caaacacttt atgtattgac agtttatagc aatactgtgc ttaactgatt 50640
tatagaaaga atatttaaaa tacttgaagt aaactatttt caagtaacat gcattctaac 50700
atatgataat actgattttt aattcttctg gcatgtgtta agactaattt ttcaggcagt 50760
ctattaagca acattaacta ttttgtcatt ttatatacat aaacataatg taacttatat 50820
ataaactctc caaataatat gaacttgtga aattttttac ataattggct gtgaggagcc 50880
aagagcatat gttttcaaaa aaatagcatt ttattttctt ttgattcata agagcaaata 50940
cttttggcac cattttatat ccagttttac tacgttttct gagatgttgt aattttttat 51000
atttcctata atttagcaaa actaaattat taaccacagt aaatctggat ccttcatcca 51060
tattttatat aggaatttca gttctgcctc ataagatagt gtatatgatt tataatcttt 51120
ttaatttata aattagaaaa atctattata atgtttagtt atttcttaga tacatttcgt 51180
aaaacttttg taaaatatga aagaatcata aagtcgtagt attttagagc taaagaggac 51240
ctttgagttt gcatttgcca gtcttctatt tagtagagtg aaaaccaaat agattttatg 51300
actgccccaa agtcatatgg ctaaataggt aacccaagac aagaactgaa gcctcctgat 51360
ttattttcaa attttcttta tccatcgtgc tgccaaataa aagaatagag agaagtcttt 51420
atgcctggtg tccaaaaaag tagaaatttg gagaaaagct aatatttttg taaggttttc 51480
taccttgata atcatgaagg atgctcatta ttcgacacaa gattgtacta taaggtttgt 51540
aattgtagaa cttgaacttt tttgtttttt tggctttttt tttgcaatag aaattatctc 51600
aatggagatt tgtttcctgc caggcagtgg ctgatgcctg taatcctaac acttagggag 51660
gccagggtgg ggaggatcac tagaggccaa gagtttgaga atagcctagg caacatagta 51720
aaactctgtc tccacaaaaa attttaaaat cagcaagaaa tgatggcaca tgcctgtagt 51780
cccagctact cgggaagcca aggtgggagg attgcttgag cccaggagtt tgaggttaca 51840
gtgacctgtg gacacagcac tgccctccag cctgagtgac atagtgagac cctgtctcaa 51900
aaaaaagaag atacatgttt cctaaatcct aattttatag ccctatattt aatggtatgt 51960
ttttgtcctc gaaacattcc taaaactttg aagtaacatt ttttaaaatg tgatacagtc 52020
attctgaagc ttttttctac ccagtaatag ttactatttg ctgaacattt tttcgatata 52080
caacatacaa ttattagaaa actcagttaa cccagagaag ttaagtaaca tgttcagtgt 52140
ataaagtcat aatgttatca gaatcaagat tgaatacatg tctttgttgg caaatcccat 52200
gcttcttttt ctgaattctc tattttcctt ctatttccaa ttgaattcta aattataaga 52260
gacatttctc tccttttggt ttctgaagca ctgttactgc tttaaaggaa aagaaataag 52320
ctatcttttt actgtttcaa atattttgct tttcctatga agacttcgag atttttgctt 52380
tctacttttt ttaactatgg aaattagttg ttttgaagct gtctttttgt aatctaagta 52440
tttgaaaatg gctttgttcc tcacatttaa ttattttatt aacaatatat tttttcttta 52500
aaaaaacttt ctaggtgagg ttaaactcag acctgaagct agaaaacgac ttctaagtta 52560
tataaatgaa gagccactag atgctaatgg gtatttcaat ttgggaatgc ttgccatgga 52620
tgacaaaaag gacaatgaag cagagatttg gatgaagaaa gccataaagt tacaagccga 52680
cttccgaagt gctttgttta atctggctct cctgtattcc cagactgcaa aggaattaaa 52740
ggctttgcca attttggagg agttactcag atactaccct gatcatatca agggcctcat 52800
tttaaaagga gacattctga tgaatcaaaa gaaagatata ctaggagcaa aaaaatgttt 52860
tgaaaggatt ttggagatgg atccaagcaa tgtgcaagga aaacacaatc tttgtgttgt 52920
ttattttgaa gaaaaagact tattaaaagc tgaaagatgc cttcttgaaa cactggcatt 52980
agcaccacat gaagaatata ttcagcgcca tttgaatata gtcagggata agatttcctc 53040
atctagtttt atagagccaa tattcccaac cagtaagatt tcaagtgtgg aaggaaagaa 53100
aattccaact gaaagtgtaa aagaaattag aggtgaatcc agacaaacac aaatagtaaa 53160
aacaagtgat aataaaagtc agtctaaatc caacaaacaa ttaggaaaaa atggagacga 53220
agagacaccc cacaaaacaa caaaagacat caaagaaatt gagaagaaaa gagttgctgc 53280
tttaaaaaga ctagaagaga ttgaacgtat tttaaatggt gaataacatt aatatttatc 53340
gtgacaatgg tatcaaagaa catcaatccg tatcatgtga ttgcttttac tgggagcttt 53400
gaaaaaaagt tcaagggttc ctaatggtca atcatgagct gccttgaagt aggatcaaaa 53460
taagattttc attaaagacc tgtattatcc caggatgtat attatgtatc gctgttttca 53520

gagtgtgggt gaatatagca gaaatattac agcggaagtg acaaatttac aacttttatt 53580
atagaaagaa ggtgtttctg gcaatgtaat ctttactgct ctcaattaaa aataattttg 53640
aggcctgaat gataatccct tgaggacaaa tccaacatgt gctggtttat tctgttaatt 53700
cccatttatt tgcctacttc atttttcttg cacctcttag aatctaacta tgaattgaaa 53760
acacttaagt aattctgttt aatcaaggga tttacactac aaaagaatgc tggctttttt 53820
tatgttgtat tccttagttg agttttagaa ggaatgcttg atgaaacatt ttaaaataag 53880
tcatgacatg ttagcttgag aatgtatttt cataattgta tacttgtttt taactttaaa 53940
tgtaattttt aatcaggtaa agtttgacac atgtatagct acatacacac atttttaatg 54000
gtgctcatat atactgtatt ttttgttgtt tagttttact tattgagagt gtcacaacat 54060
gaatcacata atcatgattt ttttttttta cttttactcc ccaaattatt catgtttctt 54120
agatcgtagt cattgagaag tcccaataac tctaaacttt tgagttataa cgtagtaaac 54180
ttctctttca tctttgtgtt agctctgtag tcttaacctg gattttaatt tttttgtttc 54240
caaagtcaca attgaattat tcttagatac cttaagccac tgaattcagt tctgtttgac 54300
tgaaagcaaa acaacgtgac agtttatttt caaacactaa cttcttgata ttttgttatg 54360
gtatatcttt ttattaaata tttattttga ctaagctttc ataaaatatt tgaagctatt 54420
ttaatcatca agtatggaaa acaaattact attgcatttt cctatatatg catatattat 54480
ggattaacca gaattgtatc atttttggcc taatgtctgg atataaaaga taattagcct 54540
actatagtat taataaattt ttcagttggt ttgggcaaat ttaaacctga aaaataggtt 54600
aaaaagtagt tacaaattaa acttactaat ttatacctga ttttttttct tgaattaaag 54660
tacattttaa atgagcttta taatacctta aaaagttggt tctaatttaa aatatgaaag 54720
ctctggctat catcctggga tagtaatttc taattatata gtatttcaaa actatatatt 54780
ttttagttcc tttgagataa ctaatttcta attatatatg tttcaaaaac catatcctgt 54840
atttttttta agaattgttt tataaatagg tcataagata caaggtctgc attagaagac 54900
ccactcttac taggttccct aaggatctgc catagatttt tttttttttt tttttttttt 54960
taggtagttt aaagcaagca ctgataccag tgggagttgg tcttgatcta ggagattctg 55020
ttaagcatcc aaaaacaatg cctaatttca gttcttaggt tatggcttgt gactccagat 55080
aaaagatgga gaatacctca tgtactgtga cttgaaaatg aattcttaaa attcttaggc 55140
tctctccatg tatctttctt aaggaaaagt ttctgagtgt gatctctctt ttgccatagt 55200
atcaagtgga gggtagttca gaaaagttaa taggaaatct tttgtgacag cagactataa 55260
tagaagtttg agtaatattt taataaattt atataattca aatgataaaa atgtatcaat 55320
gttatccaat gatttttatt aaaaaattac cttattatta 55360

<210> 2
<211> 4997
<212> DNA
<213> Homo sapiens

<400> 2
   ggggcggcgg ggaaggaccg agaggcggga ggagcagcgg ctcaggcgcc tgcaaactgg 60
   tggcctgaac gaggtagacc atgactgtgg tttcagtggc gtcactcgct gggctgctct 120
   tcctgaggtt ttcctaagcc atcccctggc ggaaccgccc ccagtttttg tccagaagtg 180
   cttatagaaa agatggctaa tattaaccta aaagaaataa ccttaatagt aggtgtggtt 240
   actgcctgct attggaacag cctcttttgt ggttttgttt ttgatgatgt ttcagcaata 300
   ctggataaca aagacttgca tccatctaca cctttaaaaa ctttatttca aaatgacttc 360
   tggggaaccc ctatgtctga ggagagaagc cacaagtctt accgtccctt aacagtattg 420
   acatttcgct taaattattt gttaagtgaa ctaaaaccaa tgtcatatca tctcctgaat 480
   atgatttttc atgctgtggt tagtgtgata tttctcaaag tatgcaaact ttttctggac 540
   aacaagagta gtgtgattgc ttctttactt tttgcagtgc acccaataca cacagaagca 600
   gtaacaggag ttgttggaag agcagaactt ttgtcatcta tcttttttct agcagctttt 660
   ttgtcatata ccagatcaaa aggaccagac aattccataa tatggactcc aattgccttg 720
   acagtgtttt tagtggctgt tgcaacatta tgtaaagaac aaggaataac agttgtagga 780
   atttgctgtg tgtatgaagt gtttattgcc caggggtata ctttgccatt actatgtact 840
   actgctggac agtttctccg tggaaagggt agcattccat tttctatgct gcagacacta 900
   gtaaaactca ttgtcttgat gttcagtaca ttattacttg ttgtgattag agtccaggtt 960
   attcaatccc aacttccagt attcaccagg tttgataacc cagctgctgt aagcccaact 1020
   cctacaaggc aactaacttt taactacctc cttcctgtga atgcttggtt gttattaaat 1080
   ccttcagagc tctgctgtga ttggaccatg ggaacaatac cacttataga gtcattacta 1140
   gatattcgaa atctggccac atttactttc ttttgttttc tggggatgtt gggagtattc 1200
   agtatcagat actctggtga ttcctccaag actggtttaa tggcgctttg tttaatggca 1260
   ttaccattta ttcctgcatc gaaccttttt tttccagttg gatttgttgt tgccgagcga 1320
   gtattatatg ttcccagcat ggggttctgt attttggtag cccatggatg gcagaaaata 1380
   tcaacaaaaa gtgtatttaa aaagctatcc tggatttgtc tgtctatggt gatactcact 1440
   cattccttaa aaacattcca cagaaattgg gattgggagt ctgaatatac attgtttatg 1500
   tcagccttga aggtaaataa aaataatgcc aaactttgga ataatgtggg tcatgctctg 1560
   gaaaatgaaa agaactttga gagagctttg aaatacttct tacaggctac ccatgttcag 1620
   ccagatgata ttggtgccca tatgaatgta ggaagaactt ataaaaattt aaatagaacc 1680
   aaagaagctg aagaatctta catgatggct aaatcactga tgcctcaaat tattcctggt 1740
   aaaaaatatg cagccagaat tgcccctaac cacctaaatg tttatatcaa tctggctaac 1800
   ctgatccgag caaatgagtc ccgactggaa gaagcagatc agctgtaccg tcaagcaata 1860
   agcatgaggc ccgacttcaa gcaggcttac attagcagag gagaattgct tttaaaaatg 1920
   aataaacctc ttaaagcaaa ggaagcatat cttaaagcac tagagctgga cagaaataat 1980
   gcagatcttt ggtacaactt ggcaattgta catattgaac ttaaagaacc aaatgaagcc 2040
   ctaaaaaact ttaatcgtgc tctggaacta aatccaaagc ataaactagc attattcaac 2100
   tctgttatag taatgcaaga atcaggtgag gttaaactca gacctgaagc tagaaaacga 2160
   cttctaagtt atataaatga agagccacta gatgctaatg ggtatttcaa tttgggaatg 2220
   cttgccatgg atgacaaaaa ggacaatgaa gcagagattt ggatgaagaa agccataaag 2280
   ttacaagccg acttccgaag tgctttgttt aatctggctc tcctgtattc ccagactgca 2340
   aaggaattaa aggctttgcc aattttggag gagttactca gatactaccc tgatcatatc 2400
   aagggcctca ttttaaaagg agacattctg atgaatcaaa agaaagatat actaggagca 2460
   aaaaaatgtt ttgaaaggat tttggagatg gatccaagca atgtgcaagg aaaacacaat 2520
   ctttgtgttg tttattttga agaaaaagac ttattaaaag ctgaaagatg ccttcttgaa 2580
   acactggcat tagcaccaca tgaagaatat attcagcgcc atttgaatat agtcagggat 2640
   aagatttcct catctagttt tatagagcca atattcccaa ccagtaagat ttcaagtgtg 2700
   gaaggaaaga aaattccaac tgaaagtgta aaagaaatta gaggtgaatc cagacaaaca 2760
   caaatagtaa aaacaagtaa taataaaagt cagtctaaat ccaacaaaca attaggaaaa 2820
   aatggagacg aagaggcacc ccacaaaaca acaaaagaca tcaaagaaat tgagaagaaa 2880
   agagttgctg ctttaaaaag actagaagag attgaacgta ttttaaatgg tgaataacat 2940
   taatatttat cgtgacaatg gtatcaaaga acatcaatcc gtatcatgtg attgctttta 3000
   ctgggagctt tgaaaaaaag ttcaagggtt cctaatggtc aatcatgagc tgccttgaag 3060
   taggatcaaa ataagatttt cattaaagac ctgtattatc ccaggatgta tattatgtat 3120
   cgctgttttc agagtgtggg tgaatatagc agaaatatta cagcggaagt gacaaattta 3180
   caacttttat tatagaaaga aggtgtttct ggcaatgtaa tctttactgc tctcaattaa 3240
   aaataatttt gaggcctgaa tgataatccc ttgaggacaa atccaacatg tgctggttta 3300
   ttctgttaat tcccatttat ttgcctactc cattttcttt gcacctctta gaatctaact 3360
   atgaattgaa aacacttaag taattctgtt taatcaaggg atttacacta caaaagaatg 3420
   ctggcttttt ttatgttgta ttccttagtt gagttttaga aggaatgctt gatgaaacat 3480
   tttaaaataa gtcatgacat gttagcttga gaatgtattt tcatgattgt atacttgttt 3540
   ttaactttaa atgtaatttt taatcaggta aagtttgaca catgtatagc tacatacaca 3600
   catttttaat ggtgctcata tatactgtat tttttgttgt ttagttttac ttattgagag 3660
   tgtcacaaca tgaatcacat aatcatgatt tttttttttt acttttactc cccaaattat 3720
   tcatgtttct tagatcgtag tcattgagaa gtcccaataa ctctaaactt ttgagttata 3780
   acgtagtaaa cttctctttc atctttgtgt tagctctgta gtcttaacct ggatttaaat 3840
   ttttttgttt ccaaagtcac aattgaatta ttcttagata ccttaagcca ctgaattcag 3900
   ttctgtttga ctgaaagcaa aacaacgtga cagtttattt tcaaacacta acttcttgat 3960
   attttgttat ggtatatctt tttattaaat atttattttg actaagcttt cataaaatat 4020
   ttgaagctat tttaatcatc aagtatggaa aacaaattac tattgcattt tcctatatat 4080
   gcatatatta tggattaacc agaattgtat catttttggc ctaatgtctg gatataaaag 4140
   ataattagcc tactatagta ttaataaatt tttcagttgg tttgggcaaa tttaaacctg 4200
   aaaaataggt taaaaagtag ttacaaatta aacttactaa tttatacctg attttttttc 4260
   ttgaattaaa gtacatttta aatgagcttt ataatacctt aaaaagttgg ttctaattta 4320
   aaatatgaaa gctctggcta tcaccctggg atagtaattt ctaattatat agtatttcaa 4380
   aactatatat tttttagttc ctttgagata actaatttct aattatatat gtttcaaaaa 4440
   ccatatcctg tatttttttt aagaattgtt ttataaatag gtcataagat acaaggtctg 4500
   cattagaaga cccactctta ctaggttccc taaggatctg ccatagattt tttttttttt 4560
   tttttttttt ttaggtagtt taaagcaagc actgatacca gtgggagttg gtcttgatct 4620
   aggagattct gttaagcatc caaaaacaat gcctaatttc agttcttagg ttatggcttg 4680
   tgactccaga taaaagatgg agaatacctc atgtactgtg acttgaaaat gaattcttaa 4740
   aattcttagg ctctctccat gtatctttct taaggaaaag tttctgagtg tgatctctct 4800
   tttgccatag tatcaagtgg agggtagttc agaaaagtta ataggaaatc ttttgtgaca 4860
   gcagactata atagaagttt gagtaatatt ttaataaatt tatataattc aaatgataaa 4920
   aatgtatcaa tgttatccaa tgatttttat taaaaaatta ccttattatt aaaaaaaaaa 4980
   aaaaaaaaaa aaaaaaa 4997

<210> 3
<211> 914
<212> PRT
<213> Homo sapiens

<400> 3

Met Ala Asn Ile Asn Leu Lys Glu Ile Thr Leu Ile Val Gly Val Val
1               5                   10                  15
Thr Ala Cys Tyr Trp Asn Ser Leu Phe Cys Gly Phe Val Phe Asp Asp
            20                  25                  30
Val Ser Ala Ile Leu Asp Asn Lys Asp Leu His Pro Ser Thr Pro Leu
        35                  40                  45
Lys Thr Leu Phe Gln Asn Asp Phe Trp Gly Thr Pro Met Ser Glu Glu
    50                  55                  60
Arg Ser His Lys Ser Tyr Arg Pro Leu Thr Val Leu Thr Phe Arg Leu
65                  70                  75                  80
Asn Tyr Leu Leu Ser Glu Leu Lys Pro Met Ser Tyr His Leu Leu Asn
                85                  90                  95
Met Ile Phe His Ala Val Val Ser Val Ile Phe Leu Lys Val Cys Lys
            100                 105                 110
Leu Phe Leu Asp Asn Lys Ser Ser Val Ile Ala Ser Leu Leu Phe Ala
        115                 120                 125
Val His Pro Ile His Thr Glu Ala Val Thr Gly Val Val Gly Arg Ala
    130                 135                 140
Glu Leu Leu Ser Ser Ile Phe Phe Leu Ala Ala Phe Leu Ser Tyr Thr
145                 150                 155                 160
Arg Ser Lys Gly Pro Asp Asn Ser Ile Ile Trp Thr Pro Ile Ala Leu
                165                 170                 175
Thr Val Phe Leu Val Ala Val Ala Thr Leu Cys Lys Glu Gln Gly Ile
            180                 185                 190
Thr Val Val Gly Ile Cys Cys Val Tyr Glu Val Phe Ile Ala Gln Gly
        195                 200                 205
Tyr Thr Leu Pro Leu Leu Cys Thr Thr Ala Gly Gln Phe Leu Arg Gly
    210                 215                 220
Lys Gly Ser Ile Pro Phe Ser Met Leu Gln Thr Leu Val Lys Leu Ile
225                 230                 235                 240
Val Leu Met Phe Ser Thr Leu Leu Leu Val Val Ile Arg Val Gln Val
                245                 250                 255
Ile Gln Ser Gln Leu Pro Val Phe Thr Arg Phe Asp Asn Pro Ala Ala
            260                 265                 270
Val Ser Pro Thr Pro Thr Arg Gln Leu Thr Phe Asn Tyr Leu Leu Pro
        275                 280                 285
Val Asn Ala Trp Leu Leu Leu Asn Pro Ser Glu Leu Cys Cys Asp Trp
    290                 295                 300
Thr Met Gly Thr Ile Pro Leu Ile Glu Ser Leu Leu Asp Ile Arg Asn
305                 310                 315                 320
Leu Ala Thr Phe Thr Phe Phe Cys Phe Leu Gly Met Leu Gly Val Phe
                325                 330                 335
Ser Ile Arg Tyr Ser Gly Asp Ser Ser Lys Thr Gly Leu Met Ala Leu
            340                 345                 350
Cys Leu Met Ala Leu Pro Phe Ile Pro Ala Ser Asn Leu Phe Phe Pro
        355                 360                 365
Val Gly Phe Val Val Ala Glu Arg Val Leu Tyr Val Pro Ser Met Gly
    370                 375                 380
Phe Cys Ile Leu Val Ala His Gly Trp Gln Lys Ile Ser Thr Lys Ser
385                 390                 395                 400

Val Phe Lys Lys Leu Ser Trp Ile Cys Leu Ser Met Val Ile Leu Thr
                405                 410                 415
His Ser Leu Lys Thr Phe His Arg Asn Trp Asp Trp Glu Ser Glu Tyr
            420                 425                 430
Thr Leu Phe Met Ser Ala Leu Lys Val Asn Lys Asn Asn Ala Lys Leu
        435                 440                 445
Trp Asn Asn Val Gly His Ala Leu Glu Asn Glu Lys Asn Phe Glu Arg
    450                 455                 460
Ala Leu Lys Tyr Phe Leu Gln Ala Thr His Val Gln Pro Asp Asp Ile
465                 470                 475                 480
Gly Ala His Met Asn Val Gly Arg Thr Tyr Lys Asn Leu Asn Arg Thr
                485                 490                 495
Lys Glu Ala Glu Glu Ser Tyr Met Met Ala Lys Ser Leu Met Pro Gln
            500                 505                 510
Ile Ile Pro Gly Lys Lys Tyr Ala Ala Arg Ile Ala Pro Asn His Leu
        515                 520                 525
Asn Val Tyr Ile Asn Leu Ala Asn Leu Ile Arg Ala Asn Glu Ser Arg
    530                 535                 540
Leu Glu Glu Ala Asp Gln Leu Tyr Arg Gln Ala Ile Ser Met Arg Pro
545                 550                 555                 560
Asp Phe Lys Gln Ala Tyr Ile Ser Arg Gly Glu Leu Leu Leu Lys Met
                565                 570                 575
Asn Lys Pro Leu Lys Ala Lys Glu Ala Tyr Leu Lys Ala Leu Glu Leu
            580                 585                 590
Asp Arg Asn Asn Ala Asp Leu Trp Tyr Asn Leu Ala Ile Val His Ile
        595                 600                 605
Glu Leu Lys Glu Pro Asn Glu Ala Leu Lys Asn Phe Asn Arg Ala Leu
    610                 615                 620
Glu Leu Asn Pro Lys His Lys Leu Ala Leu Phe Asn Ser Val Ile Val
625                 630                 635                 640
Met Gln Glu Ser Gly Glu Val Lys Leu Arg Pro Glu Ala Arg Lys Arg
                645                 650                 655
Leu Leu Ser Tyr Ile Asn Glu Glu Pro Leu Asp Ala Asn Gly Tyr Phe
            660                 665                 670
Asn Leu Gly Met Leu Ala Met Asp Asp Lys Lys Asp Asn Glu Ala Glu
        675                 680                 685
Ile Trp Met Lys Lys Ala Ile Lys Leu Gln Ala Asp Phe Arg Ser Ala
    690                 695                 700
Leu Phe Asn Leu Ala Leu Leu Tyr Ser Gln Thr Ala Lys Glu Leu Lys
705                 710                 715                 720
Ala Leu Pro Ile Leu Glu Glu Leu Leu Arg Tyr Tyr Pro Asp His Ile
                725                 730                 735
Lys Gly Leu Ile Leu Lys Gly Asp Ile Leu Met Asn Gln Lys Lys Asp
            740                 745                 750
Ile Leu Gly Ala Lys Lys Cys Phe Glu Arg Ile Leu Glu Met Asp Pro
        755                 760                 765
Ser Asn Val Gln Gly Lys His Asn Leu Cys Val Val Tyr Phe Glu Glu
    770                 775                 780
Lys Asp Leu Leu Lys Ala Glu Arg Cys Leu Leu Glu Thr Leu Ala Leu
785                 790                 795                 800
Ala Pro His Glu Glu Tyr Ile Gln Arg His Leu Asn Ile Val Arg Asp
                805                 810                 815
Lys Ile Ser Ser Ser Ser Phe Ile Glu Pro Ile Phe Pro Thr Ser Lys
            820                 825                 830
Ile Ser Ser Val Glu Gly Lys Lys Ile Pro Thr Glu Ser Val Lys Glu
        835                 840                 845
Ile Arg Gly Glu Ser Arg Gln Thr Gln Ile Val Lys Thr Ser Asn Asn
    850                 855                 860
Lys Ser Gln Ser Lys Ser Asn Lys Gln Leu Gly Lys Asn Gly Asp Glu
865                 870                 875                 880
Glu Ala Pro His Lys Thr Thr Lys Asp Ile Lys Glu Ile Glu Lys Lys

885                                     890                                     895
Arg Val Ala Ala Leu Lys Arg Leu Glu Glu Ile Glu Arg Ile Leu Asn
                900                                     905                                     910
Gly Glu

## Description

The present invention concerns a method for the in vitro diagnosis of a graft tolerant or graft non-tolerant phenotype, comprising: determining from a grafted subject biological sample an expression profile comprising TMTC3 gene, optionally measuring other parameters, and determining the presence of a graft tolerant or graft non-tolerant phenotype from said expression profile and optional other parameters, wherein said method does not comprise determining an expression profile comprising, in addition to TMTC3, the following 7 genes: BUB1B, CDC2, CHEK1, MS4A1, RAB30, RHOH, and SYNGR3. Said method may further comprise, if said subject is diagnosed as a graft non-tolerant subject, diagnosing from the expression profile if said subject is developing chronic or acute rejection.

Currently, the long-term survival of an allograft is depending on the continuous administration of immunosuppressive drugs. Indeed, an interruption of the immunosuppressive treatment generally leads to an acute or chronic rejection, particularly in case of an early or abrupt diminution.

However, long-term immunosuppressive treatments lead to severe side effects such as chronic nephrotoxicity, an increased susceptibility to opportunistic infections, and a dose-dependant increased propensity to develop virus induced malignancies (1).

Despite the difficulties encountered by many attempts to induce a persistent tolerance to allografts in human, it has been observed that some patients can maintain the tolerance to their graft without any immunosuppressive treatment (ref 2), demonstrating that a state of operational tolerance may naturally occur, even in humans.

In the case of kidney graft, the real proportion of tolerant grafted subjects may be underestimated. Indeed, although the possibility to progressively stop the immunosuppressive treatment has never been investigated, a significant proportion of kidney grafted subject accept their graft with a minimal dose of immunosuppressive drug (cortisone monotherapy, <10mg a day) (2). In addition, among patients developing post-transplantation lymphoproliferative disorders, leading to the interruption of their immunosuppressive treatment, some does not reject their graft.

Thus, a significant proportion of kidney grafted subjects might display an unsuspected, total or partial, immune operational tolerance state to their graft. It would therefore be very useful to have a method to diagnose, without any previous modification of the immunosuppressive treatment, the level of immune tolerance of grafted subjects taken individually. Indeed, this would allow for an ethically acceptable, progressive, total or partial withdrawal of immunosuppressive drugs in subject with a high enough level of graft tolerance. Although well known biological parameters are used by clinicians for the evaluation of renal function (creatinemia, proteinuria and urea serum concentrations and clearance), these parameters are not sufficient for a precise diagnosis of tolerance or rejection and most importantly, have no predictive value. Currently, only a biopsy of the grafted kidney allows, through the analysis of the presence or absence of several histological lesion types (3), for the precise evaluation of said grafted kidney functionality. However, a biopsy is an invasive examination, which is not without danger for the grafted organ, and is thus usually not performed on grafted subjects that have stable biological parameters values. In addition, the variability of the diagnosis, due to the subjectivity of the analysis, is a drawback of the histological examination of biopsies. A non-invasive accurate and reliable method of diagnosis of a graft tolerant phenotype is thus needed.

In addition, in the case of many grafted organ, when the values of standard biological parameters allow for the diagnostic of chronic rejection, the rejection process is already in progress and, although it may in certain cases be stopped, the lesions that have been induced generally cannot be reversed. Moreover, to confirm the diagnostic, a biopsy of the grafted organ is usually performed, which is, as stated before, not without danger. It would thus also be very valuable to have a non-invasive method allowing diagnosing chronic rejection at the earlier steps of the rejection process, which would permit to adapt the immunosuppressive treatment and might in some cases prevent the chronic rejection process.

Finally, a non-invasive method for an early and reliable diagnosis of a graft tolerant or non-tolerant phenotype would be very useful in clinical research, since it would allow for relatively short (6 months to 1 year), and thus less expensive, clinical trial studies.

At the present time, few genome-wide studies have been carried out in humans on the modifications of gene expression patterns after kidney transplant. In addition, these studies focused on the identification of genes implicated in graft acute or chronic rejection, and not in graft tolerance. From the comparison of the expression level of about 12000 unique genes in tolerant patients versus patients in chronic rejection, the inventors identified TMTC3 as a gene significantly differentially expressed between the two groups of patients, and that permits a reliable identification of graft-tolerant or graft non-tolerant patients among a group of grafted patients.

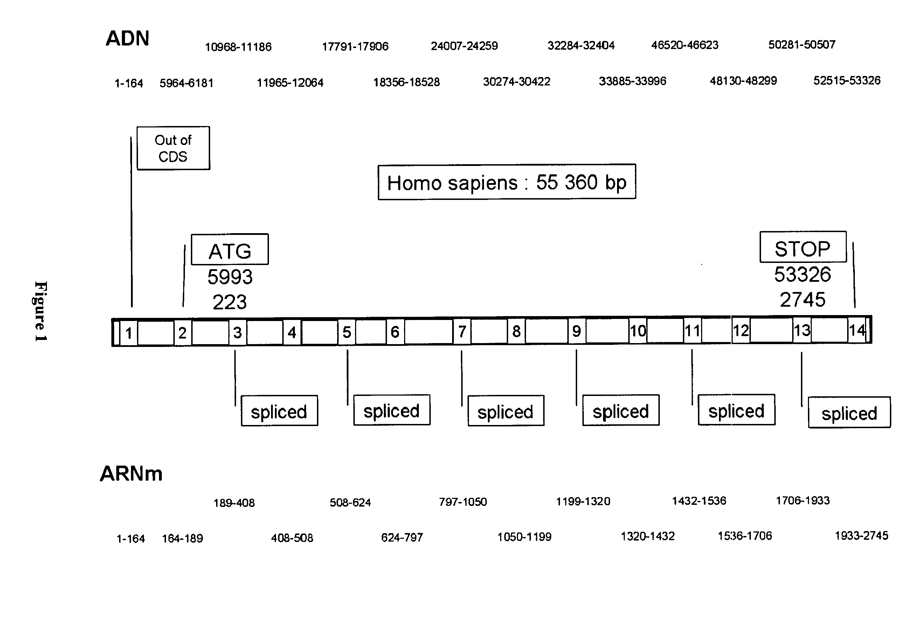

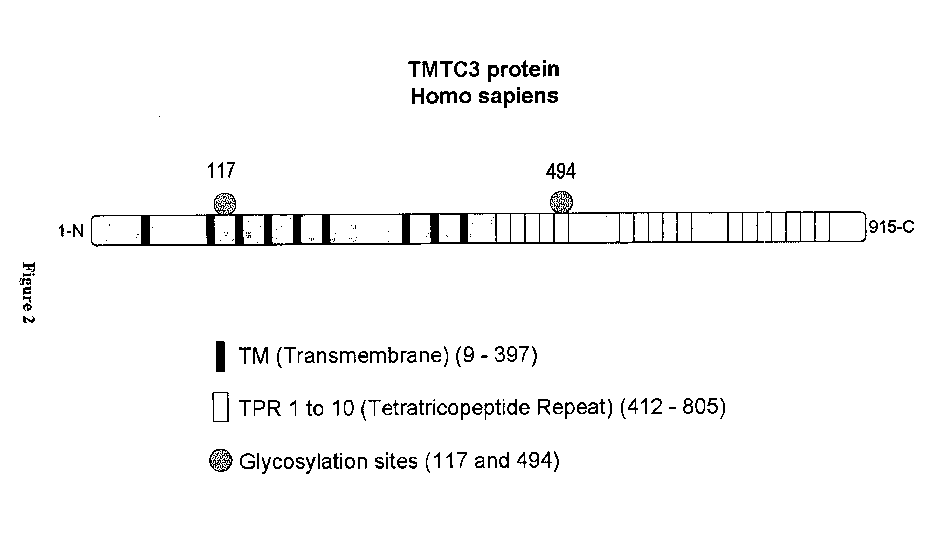

Human TMTC3 (Transmembrane and tetratricopeptide repeat containing 3), also named SMILE, is a transmembrane tetratricopeptide located on chromosome 12 (12q21.32) which function is still largely unknown. The genomic sequence of human TMTC3 gene is available in Genbank under accession number NC_000012.10, in region comprising nucleotides 87060232-87115591 of this chromosome 12 genomic contig (SEQ ID NO:1), while the mRNA and protein sequence are available under accession numbers NM_181783.2 (SEQ ID NO:2) and NP_861448.1 (SEQ ID NO:3) respectively. The structure of the nucleic sequences (genomic and mRNA) are displayed in Figure 1. The structure of TMTC3 protein with conserved domains is displayed in Figure 2.

Thanks to the identification of TMTC3 as a gene significantly differentially expressed between tolerant patients and patients in chronic rejection, it is now possible to use a very simple and non-invasive method of in vitro diagnosis of a graft tolerant or, on the contrary, a graft non-tolerant phenotype. Such a method allows for the identification of grafted subject for whom a progressive, total or partial withdrawal of immunosuppressive drugs is possible. It also permits an early diagnosis of a chronic rejection process in patients whose biological parameters levels are still normal. Moreover, the diagnosis may be performed from a blood sample, which is completely harmless for the tested grafted subject. Finally, the expression of only one gene is easily performed.

The invention thus concerns a method for the in vitro diagnosis of a graft tolerant or non-tolerant phenotype, comprising or consisting in:
(a) determining from a grafted subject biological sample an expression profile comprising, or consisting of TMTC3 gene,
(b) comparing the obtained expression profile with at least one reference expression profile, and
(c) determining the graft tolerant or graft non-tolerant phenotype from said comparison,
wherein said method does not comprise determining an expression profile comprising, in addition to TMTC3, the following 7 genes: BUB1B, CDC2, CHEK1, MS4A1, RAB30, RHOH, and SYNGR3.

The main features of 7 genes that should not all be comprised in the determined expression profile, in addition to TMTC3, are listed in the following Table 1. However, one or more of these genes may be included in the determined expression profile, provided that not all of them are included, i.e. provided that the expression profile does not comprise these 7 genes and the TMTC3 gene.

**Table 1. Main features of 7 genes not to be all included in the determined expression profile.**

| **N°** | **Symbol** | **Name** | **Accession Nb (RefSeq)** | **LLocus ID** | **Synonyms** | **UniGeneID** | **LocChr** |
|---|---|---|---|---|---|---|---|
| 1 | BUB1B | BUB1 budding uninhibited by benzimidazoles 1 homolog beta (yeast) | NM_001211 | 701 | BUB1beta, BUBR1, Bub1A MAD3L, SSK1, hBUBR1 | Hs.631699 | 15q15 |
| 2 | CDC2 | cell division cycle 2, G1 to S and G2 to M | NM_001786 .2 NM_03337 9.2 | 983 | CDC28A, CDK1, DKFZp686L 20222, MGC11119 5 | Hs.334562 | 10q21.1 |
| 3 | CHEK1 | CHK1 checkpoint homolog (S. pombe) | NM_001274 .2 | 1111 | CHK1 | Hs.24529 | 11q24-q24 |
| 4 | MS4A1 | membrane-spanning 4-domains, subfamily A, member 1 | NM_152866 .2 NM_02195 0.3 | 931 | B1, Bp35, CD20, LEU-16, MGC3969, MS4A2, S7 | Hs.438040 | 11q12 |
| 5 | RAB30 | RAB30, member RAS oncogene family | NM_014488 .3 | 27314 | Ras-related protein Rab-30 | Hs.40758 | 11q12-q14 |
| 6 | RHOH | ras homolog gene family, member H | NM_004310 .2 | 399 | ARHH, TTF | Hs.160673 | 4p13 |
| 7 | SYNGR3 | synaptogyrin 3 | NM_004209 .4 | 9143 | MGC:20003 | Hs.435277 | 16p13 |

According to the present invention, a "graft tolerant phenotype" is defined as a state of tolerance of a subject to his graft. A "state of tolerance" means that this subject (referred to as a "graft tolerant subject") does not reject his graft in the absence of an immunosuppressive treatment with a well functioning graft. In contrast, a "graft non-tolerant phenotype" refers to the absence in said subject of a state of tolerance, meaning that said subject (referred to as a "graft non-tolerant subject") would, at the time of the diagnosis, reject its graft if the immunosuppressive treatment was withdrawn. While the population of graft tolerant subjects only includes subjects in a state of tolerance to their graft, the population of graft non-tolerant subjects thus includes all other subjects and is composed of a variety of different states: patients in acute rejection, patients already suffering from obvious chronic rejection, patients at the early non symptomatic stage of chronic rejection, but also stable patients, which cannot at this time be considered as tolerant but who may later develop a graft tolerant phenotype. Indeed, it must be understood that the mechanisms of tolerance are complex and still not elucidated, and the cellular and molecular processes of tolerance induction may require a prolonged laps of time. Thus, while the population of graft tolerant subjects only includes subjects who have already reached a stable state of tolerance to their graft, the population of graft non-tolerant subjects is heterogeneous and includes all other subjects, i.e. both subjects in the process of developing acute or chronic rejection and subjects in the process of developing tolerance.

Immunosuppressive drugs that may be employed in transplantation procedures include azathioprine, methotrexate, cyclophosphamide, FK-506, rapamycin, corticosteroids, and cyclosporins. These drugs may be used in monotherapy or in combination therapies.

In the case of kidney graft, the following immunosuppressive protocols are usually used.

Subjects with primary kidney graft generally receive an induction treatment consisting of 2 injections of basiliximab (Simulect®, a chimeric murine/human monoclonal anti IL2-Rα antibody commercialized by Novartis), in association with tacrolimus (Prograf™, Fujisawa Pharmaceutical, 0.1 mg/kg/day), mycophenolate mofetil (Cellcept™, Syntex Laboratories, Inc, 2 g/day) and corticoids (1 mg/kg/day), the corticoid treatment being progressively decreased of 10 mg every 5 days until end of treatment, 3 months post transplantation.

Subjects with secondary or tertiary kidney graft, or subjects considered at immunological risk (percentage of anti-T PRA previously peaking above 25% or cold ischemia for more than 36 hours), generally receive a short course of anti-thymocyte globulin (ATG) (7 days), in addition from day 0 with mycophenolate mofetil (Cellcept™, Syntex Laboratories, Inc, 2 g/day), and corticosteroids (1 mg/kg/day), then the steroids are progressively tapered of 10 mg every 5 days until end of treatment and finally stopped around 3 months post transplantation. Tacrolimus (Prograf™, Fujisawa Pharmaceutical) is introduced in a delayed manner (at 6 days) at a dose of 0.1 mg/kg/day.

The present invention possesses two major interests:
- first, it permits to diagnose or prognose (i.e. to identify), among patients under immunosuppressive treatment, those who are tolerant to their graft and who could thus benefit from a progressive partial or total withdrawal of the immunosuppressive treatment while remaining tolerant to their graft. Due to the side effects of immunosuppressive treatments, this achievement is really crucial; and
- second, it further permits more precisely to diagnose or prognose (i.e. to identify), among patients under immunosuppressive treatment who are diagnosed by the method according to the invention as graft non-tolerant (i.e. patients that are not diagnosed as graft tolerant) but who are apparently stable in view of their still normal clinical parameters, those who are already at the early steps of acute or chronic graft rejection. Thus, the invention also permits to detect patients who would need a modified immunosuppressive treatment to prevent acute or chronic rejection at the very beginning of the rejection process. In this case, the early adaptation of the immunosuppressive treatment then favors the prevention of rejection.

A "biological sample" may be any sample that may be taken from a grafted subject, such as a serum sample, a plasma sample, a urine sample, a blood sample, a lymph sample, or a biopsy. Such a sample must allow for the determination of an expression profile comprising or consisting of the TMTC3 gene. Preferred biological samples for the determination of an expression profile include samples such as a blood sample, a lymph sample, or a biopsy. Preferably, the biological sample is a blood sample, more preferably a peripheral blood sample comprising peripheral blood mononuclear cells (PBMC). Indeed, such a blood sample may be obtained by a completely harmless blood collection from the grafted patient and thus allows for a non-invasive diagnosis of a graft tolerant or non-tolerant phenotype.

By "expression profile" is meant a group of at least one value corresponding to the expression level of the TMTC3 gene, optionally with further other values corresponding to the expression levels of other genes. Preferably, the expression profile consists of a maximum of 500, 400, 300, 200, preferably 100, 75, 50, more preferably 40, 20, 10, even more preferably 9, 8, 7, 6, 5, 4, 3, 2 or 1 distinct genes, one of which is the TMTC3 gene. In a most preferred embodiment, the expression profile consists of the TMTC3 gene only, since this expression profile has been demonstrated to be particularly relevant for assessing graft tolerance/non-tolerance.

The determination of the presence of a graft tolerant or graft non-tolerant phenotype is carried out thanks to the comparison of the obtained expression profile with at least one reference expression profile in step (b).

A "reference expression profile" is a predetermined expression profile, obtained from a biological sample from a subject with a known particular graft state. In particular embodiments, the reference expression profile used for comparison with the test sample in step (b) may have been obtained from a biological sample from a graft tolerant subject ("tolerant reference expression profile"), and/or from a biological sample from a graft non-tolerant subject ("non-tolerant reference expression profile"). Preferably, a non-tolerant expression profile is an expression profile of a subject suffering from acute or chronic rejection.

Preferably, at least one reference expression profile is a tolerant reference expression profile. Alternatively, at least one reference expression profile may be a non-tolerant reference expression profile (preferably a chronic or acute rejection profile). More preferably, the determination of the presence or absence of a graft tolerant phenotype is carried out by comparison with at least one tolerant and at least one non-tolerant (preferably acute or chronic rejection) reference expression profiles. The diagnosis (or prognostic) may thus be performed using one tolerant reference expression profile and one non-tolerant (preferably chronic or acute rejection) reference expression profile. Advantageously, to get a stronger diagnosis, said diagnosis is carried out using several tolerant reference expression profiles and several non-tolerant reference expression profiles.

The comparison of a tested subject expression profile with said reference expression profiles can be done using the PLS regression (Partial Least Square) which aim is to extract components, which are linear combinations of the explanatory variables (the genes), in order to model the variable response (eg: 0 if CR, 1 if TOL). The PLS regression is particularly relevant to give prediction in the case of small reference samples. The comparison may also be performed using PAM (predictive analysis of microarrays) statistical method. A non supervised PAM 3 classes statistical analysis is thus performed. Briefly, tolerant reference expression profiles, non-tolerant (preferably chronic rejection, or acute rejection) reference expression profiles, and the expression profile of the tested subject are subjected to a clustering analysis using non supervised PAM 3 classes statistical analysis. Based on this clustering, a cross validation (CV) probability may be calculated (CVₜₒₗ), which represents the probability that the tested subject is tolerant. In the same manner, another cross validation probability may be calculated (CVₙₒₙ₋ₜₒₗ), which represents the probability that the tested subject is non-tolerant. The diagnosis is then performed based on the CVₜₒₗ and/or CVₙₒₙ₋ₜₒₗ probabilities. Preferably, a subject is diagnosed as a tolerant subject if the CVₜₒₗ probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00, and the CVₙₒₙ₋ₜₒₗ probability is of at most 0.5, at most 0.4, at most 0.3, at most 0.25, at most 0.20, at most 0.15, at most 0.10, at most 0.05, at most 0.03, at most 0.02, at most 0.01, or even 0.00, Otherwise, said subject is diagnosed as a graft non-tolerant subject.

In addition, the method according to the invention further permits to diagnose if a graft non-tolerant subject is already in the process of developing a chronic graft rejection. Indeed, when chronic rejection reference expression profiles are used, the CVₙₒₙ₋ₜₒₗ probability is then a CV_{CR} probability, i.e. the probability that the tested subject is undergoing chronic rejection. Then, a more precise diagnosis of this graft non-tolerant subject may be performed based on the CVₜₒₗ and CV_{CR} probabilities. Preferably, a graft non-tolerant subject is diagnosed as developing a chronic rejection if the CV_{CR} probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00, and the CVₜₒₗ probability is of at most 0.5, at most 0.4, at most 0.3, at most 0.25, at most 0.20, at most 0.15, at most 0.10, at most 0.05, at most 0.03, at most 0.02, at most 0.01, or even 0.00.

Thus, in an embodiment of any method according to the invention, said method further comprises, if said subject is diagnosed as a graft non-tolerant subject, diagnosing from the expression profile if said subject is developing chronic rejection.

In addition, the method according to the invention further permits to diagnose if a graft non-tolerant subject is already in the process of developing acute graft rejection. Indeed, when acute rejection reference expression profiles are used, the CVₙₒₙ₋ₜₒₗ probability is then a CV_{AR} probability, i.e. the probability that the tested subject is undergoing acute rejection. Then, a more precise diagnosis of this graft non-tolerant subject may be performed based on the CVₜₒₗ and CV_{AR} probabilities. Preferably, a graft non-tolerant subject is diagnosed as developing acute rejection if the CV_{AR} probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00, and the CVₜₒₗ probability is of at most 0.5, at most 0.4, at most 0.3, at most 0.25, at most 0.20, at most 0.15, at most 0.10, at most 0.05, at most 0.03, at most 0.02, at most 0.01, or even 0.00.

Thus, in an embodiment of any method according to the invention, said method further comprises, if said subject is diagnosed as a graft non-tolerant subject, diagnosing from the expression profile if said subject is developing acute rejection.

More simply, when only the expression level of the TMTC3 gene is determined (i.e. the expression profile consists of the TMTC3 gene only), the comparison may be done by determining the ratio between the test sample TMTC3 expression level and the mean TMTC3 expression level of at least one no-tolerant reference expression level (preferably chronic or acute rejection expression level). If the ratio is of at least 1.5, preferably at least 1.6, at least 1.7, at least 1.8, more preferably at least 1.9, at least 2.0, still more preferably at least 2.1, at least 2.2, at least 2.3, at least 2.4, at least 2.5, at least 2.8, or at least 3.0, then the tested subject is diagnosed as tolerant. Otherwise, said tested subject is diagnosed as non-tolerant.

The expression profile may be determined by any technology known by a person skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of each gene corresponding protein.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a preferred embodiment, the expression profile is determined using quantitative PCR. Quantitative, or real-time, PCR is a well known and easily available technology for those skilled in the art and does not need a precise description.

In a particular embodiment, which should not be considered as limiting the scope of the invention, the determination of the expression profile using quantitative PCR may be performed as follows. Briefly, the real-time PCR reactions are carried out using the TaqMan Universal PCR Master Mix (Applied Biosystems). 6 µl cDNA is added to a 9 µl PCR mixture containing 7.5 µl TaqMan Universal PCR Master Mix, 0.75 µl of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI PRISM 7900 Sequence Detection System (Applied Biosystems). The number of template transcript molecules in a sample is determined by recording the amplification cycle in the exponential phase (cycle threshold or C_{T}), at which time the fluorescence signal can be detected above background fluorescence. Thus, the starting number of template transcript molecules is inversely related to C_{T}.

In another preferred embodiment, the expression profile is determined by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art, such as those described in patents or patent applications US 5,143,854 (4); US 5,288,644 (5); US 5,324,633 (6); US 5,432,049 (7); US 5,470,710 (8); US 5,492,806 (9); US 5,503,980 (10); US 5,510,270 (11); US 5,525,464 (12); US 5,547,839 (13); US 5,580,732 (14); US 5,661,028 (15); US 5,800,992 (16); WO 95/21265 (17); WO 96/31622 (18); WO 97/10365 (19); WO 97/27317 (20); EP 373 203 (21); and EP 785 280 (22); the disclosures of which are herein incorporated by reference.

In a preferred embodiment, the nucleic microarray is an oligonucleotide microarray comprising, or consisting of, one oligonucleotide specific for the TMTC3 gene. Preferably, the oligonucleotides are about 50 bases in length.

Suitable microarray oligonucleotides specific for the TMTC3 gene may be designed, based on the genomic sequence of this gene (Genbank accession number NC_000012.10, SEQ ID NO:1), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

In a particular embodiment of the above method according to the invention, the expression profile further comprises at least one of the genes from Table 2. In this case, the expression profile may comprise 1, 2, 3, 4, 5, 6, 7 or more, such as about 10, 15, 20, 25, 30 or even 40, 50, 60, 70, 80 or even the 102 genes from Table 2.

The additional gene(s) of Table 2 may be analyzed either simultaneously in the same expression profile as the TMTC3 gene, or as a distinct expression profile. More precisely, the determination of the expression levels of the additional gene(s) of Table 2 may be determined in a common same experiment as TMTC3, or in a separate experiment. In addition, the analysis of the results, in particular the comparison with at least one reference expression profile, may be done either in a single common expression profile comprising both TMTC3 and genes of Table 2, or as two distinct expression profiles comprising respectively 1) TMTC3and 2) at least one gene from Table 2 (for instance the 102 genes from Table 2).

In a particular embodiment of the second case, the method according to the invention as described above further comprises between steps (b) and (c) the steps of:
(b1) obtaining from a grafted subject biological sample an expression profile comprising, or consisting of, at least one gene (for instance 1, 2, 3, 4, 5, 6, 7 or more, such as about 10, 15, 20, 25, 30 or even 40, 50, 60, 70, 80 or even the 102 genes) from Table 2,
(b2) comparing the obtained expression profile with at least one reference expression profile,
wherein in step (c), the graft tolerant or graft non-tolerant phenotype is determined from the comparison of both step (b1) and step (b2).

Indeed, the genes displayed in following Table 2 are further genes determined by the inventors as being relevant for the appreciation of the operational tolerance state of kidney grafted patients, and may thus be used in addition to TMTC3.

**Table 2. 102 genes differentially expressed between kidney transplanted subjects that are tolerant (Tol) or in chronic rejection (CR).**

| **N°** | **Symbol** | **Name** | **Accession Nb** | **LLocus ID** | **Synonyms** | **Refseq RefSeq** | **UniGene ID** | **Locchr LocChr** |
|---|---|---|---|---|---|---|---|---|
| 1 | ADAMTS7 | a disintegrin-like and metalloprotease (reprolysin type) with thrombospondin type 1 motif, 7 | NM_014272 | 11173 | ADAM-TS7, DKFZp434H 204 | NM_014272 | Hs.16441 | 15q24.2 |
| 2 | ANPEP | alanyl (membrane) aminopeptidase (aminopeptidase N, aminopeptidase M, microsomal aminopeptidase, CD13, p150) | NM_001150 | 290 | CD13, LAP1, PEPN, gp150 | NM_001150 | Hs.1239 | 15q25-q26 |
| 3 | ANXA2 | annexin A2 | NM_004039 | 302 LPC2D, | ANX2, LIP2, LPC2, CAL1H, ANX2L4 | NM 004039 | Hs.46286 4 | 15q21-q22 |
| 4 | ANXA4 | annexin A4 | NM_001153 | 307 | ANX4 | NM_001153 | Hs.42298 6 | 2p13 |
| 5 | ARPC3B | actin related protein 2/3 complex, subunit 3B, 21kDa | AL133174 | 87171 | dJ470L14.3 | NG_002363 | 0 | 20q13.1 3 |
| 6 | BDP1 | B double prime 1. subunit of RNA Polymerase III transcription initiation factor IIIB | NM_018429 | 55814 | TFC5, TFNR, TAF3B1, KIAA1241, KIAA1689, TFIIIB90, HSA238520 , TFIIIB150 | NM_018429 | Hs.27280 8 | 5q12-q13 |
| 7 | BLK | B lymphoid tyrosine kinase | NM_001715 | 640 | MGC10442 | NM_001715 | Hs.38990 0 | 8p23-p22 |
| 8 | BUB1 | BUB1 budding uninhibited by benzimidazoles 1 homolog (yeast) | NM_004336 | 699 | 0 | NM_004336 | Hs.28747 2 | 2q14 |
| 9 | C3AR1 | complement component 3a receptor 1 | NM_004054 | 719 | AZ3B, C3AR, HNFAG09 | NM_004054 | Hs.15593 5 | 12p13.3 1 |
| 10 | C5orf13 | chromosome 5 open reading frame 13 | NM_004772 | 9315 | P311, PTZ17, D4S114, PRO1873 | NM_004772 | Hs.50874 1 | 5q22.2 |
| 11 | CCR6 | chemokine (C-C motif) receptor 6 | NM_031409 | 1235 | BN-1, CKR6, DCR2, CKRL3, DRY-6, GPR29, CKR-L3, CMKBR6, GPRCY4, STRL22, GPR-CY4 | NM_004367 | Hs.46468 | 6q27 |
| 12 | CD33 | CD33 antigen (gp67) | NM_001772 | 945 | p67, SIGLEC-3 | NM_001772 | Hs.83731 | 19q13.3 |
| 13 | CD7 | CD7 antigen (p41) | NM_006137 | 924 | GP40, TP41, Tp40, LEU-9 | NM 006137 | Hs.36972 | 17q25.2 -q25.3 |
| 14 | CENPE | centromere protein E, 312kDa | NM_001813 | 1062 | KIF10 | NM_001813 | Hs.75573 | 4q24-q25 |
| 15 | L26953 | chromosomal protein mRNA, complete cds. | L26953 | 0 | 0 | 0 | 0 | 0 |
| 16 | CLEC2 | C-type lectin-like receptor-2 | NM_016509 | 51266 | 0 | NM_016509 | Hs.40979 4 | 12p13.3 1 |
| 17 | E2F5 | E2F transcription factor 5, p130-binding | NM_001951 | 1875 | E2F-5 | NM_001951 | Hs.44790 5 | 8q21.2 |
| 18 | F2 | coagulation factor II (thrombin) | NM_000506 | 2147 | PT | NM_000506 | Hs.76530 | 11p11-q12 |
| 19 | FKBP1A | FK506 binding protein 1A, 12kDa | M80199 | 2280 | FKBP1, PKC12, PKCI2, FKBP12, PPIASE, FKBP-12, FKBP12C | NM_000801 | Hs.37463 8 | 20p13 |
| 20 | FKRP | fukutin related protein | NM_024301 | 79147 | MDC1C, LGMD2I, MGC2991, FLJ12576 | 0 | Hs.19326 1 | 19q13.3 3 |
| 21 | FLJ22222 | hypothetical protein FLJ22222 | NM_175902 | 79701 | 0 | NM 024648 | Hs.43623 7 | 17q25.3 |
| 22 | FLJ22662 | hypothetical protein FLJ22662 | BC000909 | 79887 | 0 | NM_024829 | Hs.17847 0 | 12p13.2 |
| 23 | FLRT1 | fibronectin leucine rich transmembrane protein 1 | NM_013280 | 23769 | 0 | NM 013280 | Hs.52375 5 | 11q12-q13 |
| 24 | FOX01A | forkhead box O1A (rhabdomyosarco ma) | NM_002015 | 2308 | FKH1, FKHR, FOXO1 | NM_002015 | Hs.17013 3 | 13q14.1 |
| 25 | FRAG1 | FGF receptor activating protein 1 | AF159621 | 27315 | 0 | NM_014489 | Hs.13396 8 | 11p15.5 |
| 26 | FXYD3 | FXYD domain containing ion transport regulator 3 | X93036 | 5349 | MAT8, PLML, MAT-8 | NM_005971 | Hs.30135 0 | 19q13.1 3 |
| 27 | GCKR | glucokinase (hexokinase 4) regulatory protein | NM_001486 | 2646 | GKRP | NM_001486 | Hs.89771 | 2p23 |
| 28 | GDAP1 | gangliosideinduc ed differentiationass ociated protein 1 | NM_018972 | 54332 | CMT2G, CMT2H, CMT2K, CMT4A | NM_018972 | Hs.16895 8q13.3 0 | |
| 29 | GDI1 | GDP dissociation inhibitor 1 | NM_001493 | 2664 | GDIL, MRX41, MRX48, OPHN2, XAP-4, RHOGDI, RABGD1A, RABGDIA | NM_001493 | Hs.74576 | Xq28 |
| 30 | GLRX | glutaredoxin (thioltransferase) | AF069668 | 2745 | GRX | NM_002064 | Hs.28988 | 5q14 |
| 31 | GPR32 | G protein-coupled receptor 32 | NM_001506 | 2854 | 0 | NM_001506 | Hs.24812 5 | 19q13.3 |
| 32 | GPX3 | glutathione peroxidase 3 (plasma) | NM_002084 | 2878 | 0 | NM_002084 | Hs.38679 3 | 5q23 |
| 33 | GRSP1 | GRP1-binding protein GRSP1 | XM_114303 | 23150 | KIAA1013 | XM_114303 | Hs.15886 7 | 3p14.2 |
| 34 | HLA-DOB | major histocompatibility complex, class II, DO beta | NM_002120 | 3112 | 0 | NM_002120 | Hs.1802 | 6p21.3 |
| 35 | HMGB2 | high-mobility group box 2 | NM_002129 | 3148 | HMG2 | NM_002129 | Hs.43495 3 | 4q31 |
| 36 | HNRPA1 | heterogeneous nuclear ribonucleoprotein A1 | NM_002136 /NM_031157 | 3178 | HNRNPA1 | NM_002136 | Hs.35672 1 | 12q13.1 |
| 37 | HOXA1 | homeo box A1 | NM_005522 | 3198 | HOX1F, MGC45232 | NM_005522 | Hs.67397 | 7p15.3 |
| 38 | HSPA6 | heatshock 70kDa protein 6 (HSP70B') | NM_002155 | 3310 | 0 | NM_002155 | Hs.3268 | 1q23 |
| 39 | IBSP | integrin-binding sialoprotein (bone sialoprotein, bone sialoprotein II) | NM_004967 | 3381 | BSP, BNSP, SP-II, BSP-II | NM_004967 | Hs.49215 | 4q21-q25 |
| 40 | ILK | integrin-linked kinase | NM_004517 | 3611 | P59 | NM_004517 | Hs.6196 | 11p15.5 -p15.4 |
| 41 | ILT7 | leukocyte immunoglobulinlike receptor, subfamily A (without TM domain), member 4 | NM_012276 | 23547 | LILRA4 | NM_012276 | Hs.40670 8 | 19q13.4 |
| 42 | BC017857 | cDNA clone IMAGE:4690793, with apparent retainedintron. | BC017857 | 0 | 0 | 0 | 0 | 0 |
| 43 | JAK2 | Janus kinase 2 (a protein tyrosine kinase) | NM_004972 | 3717 | 0 | NM_004972 | Hs.43437 4 | 9p24 |
| 44 | KIR2DL2 | killer cell immunoglobulinlike receptor, two domains, long cytoplasmic tail, 2 | NM_014219 | 3803 | CL-43, NKAT6, p58.2, CD158B1 | NM_014219 | Hs.27845 7 | 19q13.4 |
| 45 | KIR2DL4 | killer cell immunoglobulinlike receptor, two domains, long cytoplasmic tail, 4 | NM_002255 | 3805 | 103AS, 15.212, CD158D, KIR103, KIR103AS | NM_002255 | Hs.16608 5 | 19q13.4 |
| 46 | LAK | lymphocyte alpha-kinase | NM_025144 | 80216 | FLJ22670, KIAA1527 | NM_025144 | Hs.51275 4q26 3 | |
| 47 | LAMC2 | laminin, gamma 2 | NM_005562 | 3918 | EBR2, BM600, EBR2A, LAMB2T, LAMNB2, KALININ | NM_005562 | Hs.54451 | Xq24 |
| 48 | LNPEP | leucyl/cystinyl aminopeptidase | NM_005575 | 4012 | CAP, IRAP, FLAP | NM_005575 | Hs.43882 7 | 5q15 |
| 49 | LST1 | leukocyte specific transcript 1 | AF129756 | 7940 | B144, LST-1, D6S49E | NM_007161 | Hs.43606 6 | 6p21.3 |
| 50 | LTBP3 | latent transforming growth factor beta binding protein 3 | AF011407 | 4054 | LTBP2, DKFZP586 M2123 | NM_021070 | Hs.28901 9 | 11q12 |
| 51 | MARCO | macrophage receptor with collagenous structure | AF035819 | 8685 | SCARA2 | NM_006770 | Hs.67726 | 2q12-q13 |
| 52 | MMP24 | matrix metalloproteinas e 24 (membrane-inserted) | NM_006690 | 10893 | MMP25, MT5-MMP | NM 006690 | Hs.21258 1 | 20q11.2 |
| 53 | MS4A6A | membrane-spanning 4-domains, subfamily A, member 6A | NM_022349 | 64231 | CDA01, MS4A6, 4SPAN3, CD20L3, 4SPAN3.2. MGC22650 | NM_022349 | Hs.37161 2 | 11q12.1 |
| 54 | MYL9 | myosin, light polypeptide 9, regulatory | J02854 | 10398 | LC20, MLC2, MRLC1, MYRL2, MGC3505 | NM_006097 | Hs.43381 4 | 20q11.2 3 |
| 55 | MYL9 | myosin, light polypeptide 9, regulatory | BC002648 | 10398 | LC20, MLC2, MRLC1, MYRL2, MGC3505 | NM_006097 | Hs.43381 4 | 20q11.2 3 |
| 56 | MYST4 | MYST histone acetyltransferase (monocytic leukemia) 4 | NM_012330 | 23522 | qkf, MORF, MOZ2, KIAA0383, querkopf | NM_012330 | Hs.27590 | 10q22.2 |
| 57 | NCF1 | neutrophil cytosolic factor 1 (47kDa, chronic granulomatous disease, autosomal 1) | AF330627 | 4687 | NOXO2, p47phox | NM_000265 | Hs.1583 | 7q11.23 |
| 58 | NFATC2 | nuclear factor of activated T-cells, cytoplasmic, calcineurin-dependent 2 | NM_012340 | 4773 | NFAT1, NFATP | NM 012340 | Hs.35632 1 | 20q13.2 -q13.3 |
| 59 | NOTCH2 | Notch homolog 2 (Drosophila) | NM_024408 | 4853 | hN2 | NM_024408 | Hs.8121 | 1p13-p11 |
| 60 | NPC2 | Niemann-Pick disease, type C2 | BC002532 | 10577 | HE1, NP-C2, MGC1333 | NM_006432 | Hs.43322 2 | 14q24.3 |
| 61 | OSM | oncostatin M | NM_020530 | 5008 | MGC20461 | NM_020530 | Hs.24815 6 | 22q12.2 |
| 62 | PGRMC1 | progesterone receptor membrane component 1 | NM_006667 | 10857 | MPR, HPR6.6 | NM_006667 | Hs.90061 | Xq22-q24 |
| 63 | PIP5K2B | phosphatidylinosi tol4phosphate 5kinase, type II, beta | NM_003559 | 8396 | Pip4k2B, PIP5KIIB | NM_003559 | Hs.29107 0 | 17q21.2 |
| 64 | PLCB3 | phospholipase C, beta 3 (phosphatidylinos itol-specific) | NM_000932 | 5331 | 0 | NM_000932 | Hs.43713 7 | 11q13 |
| 65 | PLEKHA3 | pleckstrin homology domain containing, family A (phosphoinositide binding specific) member 3 | AF286162 | 65977 | FAPP1, FLJ20067 | NM_019091 | Hs.41086 | 2q31.3 |
| 66 | PPP1R15A | protein phosphatase 1, regulatory (inhibitor) subunit 15A | NM_014330 | 23645 | GADD34 | NM_014330 | Hs.76556 | 19q13.2 |
| 67 | PRCP | prolylcarboxypept idase (angiotensinase C) | NM_005040 | 5547 | PCP, HUMPCP | NM_005040 | Hs.31408 9 | 11q14 |
| 68 | PSME3 | proteasome (prosome, macropain) activator subunit 3 (PA28 gamma; Ki) | NM_176863 | 10197 | Ki, PA28G. REG-GAMMA, PA28-gamma | NM_005789 | Hs.15297 8 | 17q21 |
| 69 | PTGDS | prostaglandin D2 synthase 21kDa (brain) | M61900 | 5730 | PDS, PGD2, PGDS, PGDS2 | NM_000954 | Hs.44642 9 | 9q34.2-q34.3 |
| 70 | RAD52B | RAD52 homolog B (S. cerevisiae) | BC038301 | 201299 | MGC33977 | NM 145654 | Hs.19441 1 | 17q11.2 |
| 71 | RET | ret protooncogene (multiple endocrine neoplasia and medullary thyroid carcinoma 1, Hirschsprung disease) | NM_020975 | 5979 | PTC, MTC1, HSCR1, MEN2A, MEN2B, MEN2B RET51, CDHF12 | NM_000323 | Hs.35032 1 | 10q11.2 |
| 72 | RGL | RalGDS-like | NM_015149 gene | 23179 | KIAA0959 | NM_015149 | Hs.79219 | 1q25.2 |
| 73 | RTN2 | reticulon 2 | NM_005619 | 6253 | NSP2, NSPL1 | NM 005619 | Hs.47517 | 19q13.3 2 |
| 74 | SDHB | succinate dehydrogenase complex, subunit B, iron sulfur (Ip) | NM_003000 | 6390 | IP, SDH, SDH1, SDHIP | NM_003000 | Hs.64 | 1p36.1-p35 |
| 75 | SELP | selectin P (granule membrane protein 140kDa, antigen CD62) antigen CD62) | NM_003005 | 6403 | CD62, GRMP, PSEL, CD62P, GMP140, PADGEM | NM 003005 | Hs.73800 | 1q22-q25 |
| 76 | XM 10624 6 | similar to Heat shock protein HSP 90-alpha (HSP 86)(LOC152918), m RNA. | XM_106246 | 0 | 0 | 0 | 0 | 0 |
| 77 | AY032883 | similar to annexin II receptor | AY032883 | 0 | 0 | 0 | 0 | 0 |
| 78 | XM 09390 2 | similar to Immunoglobulinbinding protein 1(CD79a-binding protein 1)(B cell signal transduction moleculealpha 4) (Alpha 4 protein) (LOC166496), mRNA. | XM_093902 | 0 | 0 | 0 | 0 | 0 |
| 79 | XM 16694 1 | similar to Mitochondrial import receptor subunit TOM20homolog (Mitochondrial 20 kDa outer membrane protein) (Outermitochondr ial membrane receptor Tom20) (LOC220368), mRNA. | XM_166941 | 0 | 0 | 0 | 0 | 0 |
| 80 | XM 09277 2 | similar to dJ760C5.1 (exon similar toABCC7(ATP-binding cassette, sub-family C (CFTR/MRP),me mber 7))(LOC164389), mRNA. | XM_092772 | 0 | 0 | 0 | 0 | 0 |
| 81 | XM 16714 6 | similar to EPIDIDYMAL SECRETORY GLUTATHIONE PEROXIDASEP RECURSOR (EPIDIDYMIS-SPECIFIC GLUTATHIONE PEROXIDASE-LIKE PROTEIN)(EGLP )(LOC221579), mRNA. | XM_167146 | 0 | 0 | 0 | 0 | 0 |
| 82 | SIRT1 | sirtuin(silent mating type information regulation homolog) 1 (S. cerevisiae) | NM_012238 | 23411 2 | SIR2L1 | NM_012238 | Hs.31176 | 10q22.1 |
| 83 | SLC29A2 | solute carrier family 29 (nucleoside transporters), member 2 | NM_001532 | 3177 | ENT2, DER12, HNP36 | NM_001532 | Hs.32951 | 11q13 |
| 84 | SMS | spermine synthase | AD001528 | 6611 | SpS, SPMSY | NM_004595 | Hs.44903 2 | Xp22.1 |
| 85 | SPTLC2 | serine palmitoyltransfer ase, long chain base subunit 2 | AF111168 | 9517 | LCB2, SPT2, KIAA0526 | 0 | Hs.59403 | 14q24.3 -q31 |
| 86 | ST13 | suppression of tumorigenicity 13 (colon carcinoma) (Hsp70 interacting protein) | BC015317 | 6767 | HIP, HOP, P48, SNC6, HSPABP, FAM10A1, HSPABP1, PRO0786 | NM_003932 | Hs.37719 9 | 22q13.2 |
| 87 | STIM1 | stromal interaction molecule 1 | NM_003156 | 6786 | GOK, D11S4896E | NM 003156 | Hs.74597 | 11p15.5 |
| 88 | STRBP | spermatid perinuclear RNA binding protein | NM_018387 | 55342 | SPNR, MGC3405, FLJ11307, FLJ14223, FLJ14984, MGC21529, DKFZp434N 214 | NM_018387 | Hs.28765 9 | 9q33.3 |
| 89 | SULT1B1 | sulfotransferase family, cytosolic, 1 B, member 1 | NM_014465 | 27284 | ST1B2, SULT1B2, MGC13356 | NM_014465 | Hs.12974 2 | 4q13.3 |
| 90 | TAF1C | TATA box binding protein (TBP)-associated factor, RNA polymerase I, C, 110kDa | NM_005679 | 9013 | SL1, TAFI95, TAFI110, MGC:39976 | NM 005679 | Hs.15302 2 | 16q24 |
| 91 | TALDO1 | transaldolase 1 | AF058913 | 6888 | TAL, TAL-H, TALDOR | NM 006755 | Hs.43867 8 | 11p15.5 -p15.4 |
| 92 | TCTEL1 | t-complex-associated-testis-expressed 1-like 1 | NM_006519 | 6993 | CW-1, tctex-1 | NM_006519 | Hs.26694 0 | 6q25.2-q25.3 |
| 93 | TERA | TERA protein | NM_021238 | 58516 | 0 | NM_021238 | Hs.35622 3 | 12p11 |
| 94 | TIMM17A | translocase of inner mitochondrial membrane 17 homolog A (yeast) | AF106622 | 10440 | TIM17, TIM17A | NM_006335 | Hs.20716 | 1q32.1 |
| 95 | TLN1 | talin 1 | NM_006289 | 7094 | TLN, KIAA1027 | NM_006289 | Hs.37500 9p13 1 | |
| 96 | TPM1 | tropomyosin 1 (alpha) | NM_000366 | 7168 | CMH3, TMSA | NM 000366 | Hs.13389 2 | 15q22.1 |
| 97 | TRAF5 | TNF receptorassociat ed factor 5 | U69108 | 7188 | RNF84, MGC:39780 | NM_0004619 | Hs.38568 5 | 1q32 |
| 98 | UHRF1 | ubiquitin-like, containing PHD and RING finger domains, 1 | NM_013282 | 29128 | Np95, ICBP90, RNF106, FLJ21925 | NM 013282 | Hs.10810 6 | 19p13.3 |
| 99 | WNT16 | wingless-type MMTV integration site family, member 16 | NM_016087 | 51384 | 0 | NM_016087 | Hs.27237 5 | 7q31 |
| 100 | YPEL2 | yippee-like (Drosophila) | XM_371070 | 388403 | FKSG4 | XM_371070 | Hs.36867 2 | 17q23.2 |
| 101 | YWHAH | tyrosine 3-monooxygenase/ tryptophan 5-monooxygenase activation protein, eta polypeptide | BC003047 | 7533 | YWHA1 | NM_003405 | Hs.22675 5 | 22q12.3 |
| 102 | ZDHHC9 | zinc finger, DHHC domain containing 9 | NM_016032 | 51114 | CGI-89, ZNF379 | NM_016032 | Hs.27435 1 | 9 |

In a particular embodiment of a method according to the invention, said method may further comprise determining from a biological sample of the subject at least one additional parameter useful for the diagnosis. Such "parameters useful for the diagnosis" are parameters that cannot be used alone for a diagnosis but that have been described as displaying significantly different values between tolerant grafted subjects and subjects in chronic or acute rejection and may thus also be used to refine and/or confirm the diagnosis according to the above described method according to the invention. They may notably be selected from:
- standard biological parameters specific for said subject grafted organ type,
- phenotypic analyses of peripheral blood mononuclear cells (PBMC), and
- qualitative and/or quantitative analysis of PBMC immune repertoire.

According to the invention, "standard biological parameters specific for said subject grafted organ type" means biological parameters that are usually used by clinicians to monitor the stability of grafted subjects status and to detect graft rejection. These standard biological parameters specific for said subject grafted organ type usually comprise serum or plasma concentrations of particular proteins, which vary depending on the grafted organ type. However, these standard biological parameters specific for said subject grafted organ type are, for each organ type, well known of those skilled in the art.

For instance, standard biological parameters specific for kidney include serum or plasma urea and creatinine concentrations. In a healthy subject, the serum creatinine concentration is usually comprised between 40 to 80 µmol/L for a woman and 60 to 100 µmol/L for a man, and the serum urea concentration between 4 to 7 mmol/L.

For instance, for liver transplantation, standard biological parameters include serum or plasma concentrations of gamma glutamyl transpeptidase (GOT), aspartate aminotransferase (AST), alanine aminotransferase (ALT), lactate dehydrogenase (LDH), and bilirubin (total or conjugated).

These standard biological parameters have the advantage of being easily measurable from a blood sample, but are not sufficient to establish a precise graft tolerant or non-tolerant diagnosis, and are also not enough sensitive to allow an early chronic rejection diagnosis. However, when combined with the determination of an expression profile according to the present invention, the resulting method according to the invention makes it possible to detect graft tolerant subject whose immunosuppressive treatment could be progressively decreased, as well as apparently stable patients (relative to their biological parameters) who are potentially actually on the verge of chronic rejection.

The phenotypic analyses of peripheral blood mononuclear cells (PBMC) may comprise various types of phenotypic analysis. In particular they may comprise:
- measuring the percentage of CD4⁺ CD25⁺ T cells in peripheral blood lymphocytes, which may be performed by any technology known in the art, in particular by flow cytometry using labelled antibodies specific for the CD4 and CD25 molecules. Preferably, the percentage of CD4⁺ CD25⁺ T cells in peripheral blood lymphocytes of a tolerant subject is not statistically different from that of a healthy volunteer, whereas it is significantly lower (p < 0.05) in a non-tolerant grafted subject (23).
- determining the cytokine expression profile of T cells, which may be performed using any technology known in the art, including quantitative PCR and flow cytometry analysis. Preferably, the oligoclonal Vβ families of a non-tolerant grafted subject express increased levels compared to a healthy volunteer of TH1 or TH2 effector molecules, including interleukin 2 (IL-2), interleukin 8 (IL-8), interleukin 10 (IL-10), interleukin 13 (IL-13), transforming growth factor beta (TGF-β), interferon gamma (IFN-y) and perforin, whereas oligoclonal Vβ families of a tolerant grafted subject do not express increased levels of those effector molecules compared to a healthy volunteer (2).

The analysis of PBMC immune repertoire consists advantageously in the qualitative and quantitative analysis of the T cell repertoire (2), such as the T cell repertoire oligoclonality and the level of TCR transcripts or genes.

The T cell repertoire oligoclonality may be determined by any technology enabling to quantify the alteration of a subject T cell repertoire diversity compared to a control repertoire. Usually, said alteration of a subject T cell repertoire diversity compared to a control repertoire is determined by quantifying the alteration of T cell receptors (TCR) complementary determining region 3 (CDR3) size distributions. In a healthy subject, who can be considered as a controle repertoire, such a TCR CDR3 size distribution displays a Gaussian form, which may be altered in the presence of clonal expansions due to immune response, or when the T cell repertoire diversity is limited and reaches oligoclonality.

The level of TCR expression at the genomic, transcriptionnal or protein level is preferably determined independently for each Vβ family by any technology known in the art. For instance, the level of TCR transcripts of a particular Vβ family may be determined by calculating the ratio between these Vβ transcripts and the transcripts of a control housekeeping gene, such as the HPRT gene. Preferably, in a graft tolerant subject, a significant percentage of Vβ families display an increase in their transcript numbers compared to a normal healthy subject.

An example of methods to analyze T cell repertoire oligoclonality and/or the level of TCR transcripts, as well as scientific background relative to T cell repertoire, are clearly and extensively described in WO 02/084567 (24), which is herein incorporated by reference. Preferably, a graft tolerant subject, as well as a subject in chronic or acute rejection, displays a T cell repertoire with a significantly higher oligoclonality than a normal healthy subject.

Such additional parameters may be used to confirm the diagnosis obtained using the expression profile comprising or consisting of the TMTC3 gene. For instance, when the subject is a kidney grafted subject, certain values of the standard biological parameters may confirm a graft non-tolerant diagnosis: if the serum concentration of urea is superior to 7 mmol/L or the serum concentration of creatinine is superior to 80 µmol/L for a female subject or 100 µmol/L for a male subject, then the tested subject is diagnosed as not tolerant to his graft.

In a preferred embodiment of any above described in vitro diagnosis method according to the invention, said subject is a kidney transplanted subject. According to the invention, a "kidney transplanted subject" is a subject that was grafted with a non syngeneic, including allogenic or even xenogenic, kidney. Said kidney transplanted subject may further have been grafted with another organ of the same donor providing the kidney. In particular, said kidney transplanted subject may further have been grafted with the pancreas, and optionally a piece of duodenum, of the kidney donor.

In another preferred embodiment of any above described in vitro diagnosis method according to the invention, said subject is a liver transplanted subject. According to the invention, a "liver transplanted subject" is a subject that was grafted with a non syngeneic, including allogenic or even xenogenic, liver. Said liver transplanted subject may further have been grafted with another organ of the same donor providing the liver.

The invention is also drawn to a method of treatment of a grafted subject, comprising:
(a) determining from a subject biological sample the presence of a graft tolerant or graft non-tolerant phenotype using a method according to the invention, and
(b) adapting the immunosuppressive treatment in function of the result of step (a).

Said adaptation of the immunosuppressive treatment may consist in:
- a reduction or suppression of said immunosuppressive treatment if the subject has been diagnosed as graft tolerant, or
- a modification of said immunosuppressive treatment if the subject has been diagnosed as developing a chronic or acute rejection.

Having generally described this invention, a further understanding of characteristics and advantages of the invention can be obtained by reference to certain specific examples and figures which are provided herein for purposes of illustration only and are not intended to be limiting unless otherwise specified.

### DESCRIPTION OF THE DRAWINGS

***Figure 1******. Structure of TMTC3 genomic DNA and mRNA.*** Corresponding bases of the genomic DNA and mRNA sequences (Genbank accession numbers NC_000012.10 and NM_181783.2, SEQ ID NO:1 and 2 respectively) forming exons included in the mRNA sequence are displayed. Exons that may be spliced are indicated.The initiation ATG codon and STOP codon are also displayed.
***Figure 2******. Structure of TMTC3 protein.*** Parts of the amino acid sequence implicated in the transmembrane domain or corresponding to tetratricopeptide repeats, as well as glycosylation sites are indicated.

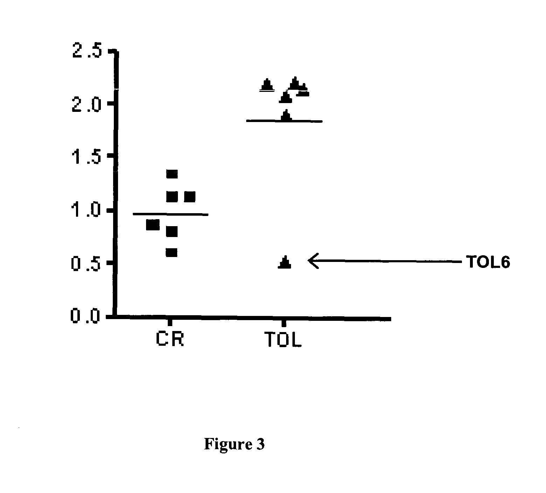

**Figure 3****. *Significant gene expression of TMTC3 in 6 tolerant patients (TOL1-6) and 6 patients in chronic rejection (CRI-6).*** A t-test, an Anova and a Kruskal-Wallis tests were performed on the 33 genes found the most accumulated by quantitative PCR. According to these tests, the TMTC3 gene was found to be highly significant between TOL and CR patients (p<0.05). The TOL6 patient is indicated.

### EXAMPLE 1

### Analysis of drug-free operational immune tolerance in human kidney graft recipients by gene expression profiling

### Patients, Materials and Methods

### Patient Selection

Peripheral blood samples were collected from 43 various adult renal transplant patients groups (tolerant patients, patients with chronic rejection, and patients with stable graft function under immunosuppression; Table 3) and 14 normal adult controls. The protocol was approved by an Ethical Committee and all patients signed a written informed consent before inclusion. Samples were separated into Training-group (analysed by microarray) and Test-group (analysed by real-time quantitative PCR) cohorts containing patient with different clinical phenotypes. Apart from tolerant patients for whom biopsy was refused by the Hospital Ethical Committee, all other patients had biopsy-confirmed clinical phenotypes.

**Table 3. Demographic summary of patient groups (Median and range).**

| | **Training Groups** | | | **Test Groups** | | | |
|---|---|---|---|---|---|---|---|
| | **TOL** | **CR** | **Normal** | **TOL-Test** | **CR-Test** | **Stable** | **Test-N** |
| Number | 5 | 11 | 8 | 6 | 6 | 7 | 14 |
| | 67 | 56 | 23 | 38.5 | 57.5 | 54 | 46 |
| Age (years) | 58-73 | 28-75 | 11-27 | 25-74 | 52-59 | 31-72 | 30-66 |
| % Male | 80% | 63.60% | 37.5% | 66% | 66% | 42.8% | 0% |
| Time post-Transplant (mo) | 178 | 59 | NA | 137 | 98 | 65 | NA |
| | | | | | | | |
| | 108-360 | 20-158 | | 56-372 | 42-158 | 23-236 | |
| Serum Creatinine (µM/l) | 122 | 244 | NA | 109 | 280.5 | 104 | NA |
| | | | | | | | |
| | 82-139 | 127-492 | | 82-139 | 127-492 | 68-147 | |
| Proteinuria per day (g/24h) | 0.83 | 1.93 | NA | 0.225 | 2.71 | 0.1 | NA |
| | 0-1.28 | 0.34-11.75 | | 0.0-0.93 | 0.56-11.75 | 0-0.25 | |
| Prior AR | 20% | 36% | NA | 33% | 16.6% | 14.3% | NA |
| Prior CA | 20% | 0% | NA | 17% | 0% | 0% | NA |
| Prior CMV | 0% | 27% | NA | 0% | 16.6% | 28.6% | NA |
| HLA incompatibi lies | 3.2 | 3 | NA | 3 | 2 | 4 | NA |
| | | | | | | | |
| | 03-4 | 01-5 | | 0-4 | 01-5 | 0-4 | |

| | | | | | | | |
|---|---|---|---|---|---|---|---|
| TOL - Tolerance; CA - Cancer, CR - Chronic Rejection; STA - Stable function; NA - Not Applicable. | | | | | | | |

To generate informative biomarkers by microarray for operational tolerance, **Training-group** samples (n=24) were chosen from 3 clinical phenotypes:
***1) Immunosuppressive drug-free, operationally tolerant (T, n=5)*:** patients with long-term stable graft function without any immunosuppression for at least 2 years (mean duration drug-free= 8.8+/-4.9 years). Stable graft function was defined as stable Cockcroft calculated creatinine clearance > 60 mls/min/1.73m2 with absent or low grade proteinuria (<1.5g/day) (2). The clinical and biological characteristics of these patients have been described in detail previously (25) and the most relevant demographic and clinical data of the entire population studied are summarized in Table 4.
***2) Chronic rejection (C, n*=*11)*:** All patients had a progressive degradation of their renal function (creatinine clearance < 60 mls/min/1.73m2 and/or proteinuria >1.5g/day) and histological signs of vascular chronic rejection defined as endarteritis and allograft glomerulopathy with basal membrane duplication. Four out of 11 patients were on dialysis due to irreversible loss of graft function, and patients from this group had completely stopped their immunosuppressive treatment for 1.5+/-0.5 years. Demographic and clinical data of these patients are shown in Table 4.
***3) Age-matched healthy volunteers (N=8)*** were included as controls. They all had a normal blood formula and no infectious or other concomitant pathology for at least 6 months prior to the study (Table 4).

To allow for validation of the discovered biomarkers for operational tolerance, an independent, blinded **Test-group** of samples (N=53) from 4 different phenotypes were examined by expression profiling using real-time PCR. The nomenclature and definitions of these different test-group cohorts are as follows:
***1) Immunosuppressive drug-free operationally tolerant test-group (TOL; N=6)*:** all new patients shared the same clinical and pathological criteria as described above (Table 4). All stopped their immunosuppression for non-adherence reasons.
***2) Chronic rejection test-group (CR, N=6)*.** all new patients shared the same clinical and pathological criteria as described above (Table 4).
***3) Long-term stable test-group (STA, N=7)*:** patients with stable kidney graft function at > 5 years post-transplantation while under mycophenolate mofetil or azathioprine, and maintenance steroids with or without an associated calcineurin inhibitor.
***4) Age-matched healthy volunteers (N, N=6).*** They all had a normal blood formulae and no infectious or other concomitant pathology for at least 6 months prior to the study.

Demographic and clinical data for all these patients are shown in Table 4.

### Microarray Experiments

Ten milliliter of peripheral blood was collected in EDTA tubes. Peripheral Blood Mononuclear Cells (PBMC) were separated on a Ficoll layer (Eurobio, Les Ulis, France) and frozen in Trizol® reagent (Invitrogen, Life technologies, California). To obviate gene expression bias based on sample collection methods, whole blood from some patients was directly tested. RNA was extracted according to the manufaturer protocol. cDNA microarrays, containing ∼32,000 cDNA clones (12,400 known unique genes) were processed using 2 µg RNA in each channel against a "common reference" RNA pool. Significance Analysis of Microarray (SAM) 2-class was used to determine significant differential gene expression between each patient group. The Cluster program (26) was used to identify gene patterns and clusters. Enrichment of functional gene classes was identified using Expression Analysis Systematic Explorer (EASE); *http:llapps1.niaid.nih.gov*/*david*/*)* and by hypergeometric enrichment analysis. Predictive analysis of Microarray or PAM class prediction (27) was used to determine the "expression phenotypes" of the unidentified, independent test group samples.

### Quantitative Real-time PCR gene expression validation

PCR primers and probes were designed to the TMTC3 gene and GAPDH, the normalizing housekeeping genes. Amplified and total RNA (100 ng) was subjected to real-time RT-PCR analysis. Quantitative PCR was performed in triplicate in an Applied Biosystems GenAmp 7700 sequence detection system (Applied Biosystems, Foster City, CA).

### Statistics

Wilcoxon rank sum test (p<0.05 used for significance), logistic regression and Pearson's correlation test (expressed as R2) were run on the clinical data.

### Results

### Biomarker Discovery using microarray experiments

Microarray analysis using a minimal gene-set of 59 transcripts representing 49 clinically relevant unique genes was performed on 24 training-group peripheral blood samples (5 T, 11 C and 8N).

Among these genes, the TMTC3 gene is over expressed in tolerant patients compared to patients in chronic rejection, and also compared to normal blood (see Table 4).

**Table 4. Expression of TMTC3 differentiates tolerance (TOL), chronic rejection (CR) and normal blood (N) in microarray experiments.**

| | **TOL vs. N** | **TOL vs. CR** |
|---|---|---|
| TMTC3 | 4.37 | 2.53 |

### "Prediction of a potential tolerant state in stable transplant patients using RT-PCR with TMTC3 gene

Quantitative RT-PCR on the TMTC3 gene from the tolerance microarray dataset and GAPDH were performed in triplicate on RNA extracted from the PBMC of 6 independent TOL-Test patients (TOL1-TOL6) and 6 independent CR-Test patients (CR1-CR6), none of whom were included in microarray analysis as well as from the PBMC of 6 healthy individuals. Seven stable transplant patients (STA1-STA7) were also analysed by QPCR. To exclude biases due to the amplification of the RNA for the microarray analysis, these PCR experiments were performed on non-amplified RNA extracted from the PBMC of the patients.

The results confirm that TMTC3 is overexpressed in tolerant patients compared to patients in chronic rejection and also compared to normal blood. The ratios are provided in following Table 5.

**Table 5. Expression of TMTC3 differentiates tolerance (TOL), chronic rejection (CR) and normal blood (N) in quantitative RT-PCR experiments.**

| | **TOL vs. N** | **TOL vs. CR** |
|---|---|---|
| TMTC3 | 4 | 2.5 |

More precisely, the TMTC3 gene was found statistically significant for the tolerance group when compared to the CR group (p< 0.005) (see Figure 3). These results were obtained by applying a t-test, an anova and a Kruskal-Wallis tests on the 33 genes found the most accumulated by quantitative PCR.

In particular, using TMTC3 expression levels in a cross-validated PAM two class analysis permitted to blindly correctly classify the tolerant and rejecting patients, with a single misclassification (TOL6 as CR). Indeed, patient TOL6 corresponds in Figure 3 to the patient showing a low expression level of TMTC3 in the TOL column. Figure 3 shows that this patient is clearly distinguishable from other TOL patients and clusters with CR patients when TMTC3 expression levels are analyzed.

Interestingly, although TOL6 fulfilled the full clinical description of operationally tolerance, 2 years prior to and at the time of harvesting, 6 months after testing, a decline in his renal function was observed (creatinemia: 165µm/l, proteinuria: 1g/day), with demonstration of anti-donor class II (anti-HLA DR4) antibodies.

Thus, the "misclassification" of patient TOL6 as a CR patient actually most probably corresponds to an early diagnosis of this patient rejection, before any clinical rejection symptom.

### Conclusion

The identification of the blood biomarker TMTC3 (SMILE) could lead to the development of a simple and minimally invasive blood test, which could be easily applied in the clinic.

Indeed, TMTC3 expression levels alone, in blood as well as in graft in transplantation, offers a diagnostic of tolerance, acute and chronic rejection. Its may thus be used as a diagnostic and prognostic marker, thereby enabling the early detection of operational tolerance, chronic and acute rejection in patients with a stable graft and under immunosuppression.

Detection of this biomarker will thus allow adapting or decreasing the treatment of these patients, avoiding rejection and/or secondary effects of immunosuppression.

### BIBLIOGRAPHY

1. Dantal J, Hourmant M, Cantarovich D, Giral M, Blancho G, Dreno B, Soulillou JP. 1998. Effect of long-term immunosuppression in kidney-graft recipients on cancer incidence: randomised comparison of two cyclosporin regimens. Lancet. 351:623-8
2. Brouard S, Dupont A, Giral M, Louis S, Lair D, Braudeau C, Degauque N, Moizant F, Pallier A, Ruiz C, Guillet M, Laplaud D, Soulillou JP. 2005. Operationally tolerant and minimally immunosuppressed kidney recipients display strongly altered blood T-cell clonal regulation. Am. J. Transplant. 5(2):330-340
3. Nankivell BJ, Borrows RJ, Fung CL, O'Connell PJ, Allen RD, Chapman JR. 2003. The natural history of chronic allograft nephropathy. N. Engl. J. Med. 349 (24):2326-33
4. US 5,143,854;
5. US 5,288,644;
6. US 5,324,633;
7. US 5,432,049;
8. US 5,470,710;
9. US 5,492,806;
10. US 5,503,980;
11. US 5,510,270;
12. US 5,525,464;
13. US 5,547,839;
14. US 5,580,732;
15. US 5,661,028;
16. US 5,800,992;
17. WO 95/21265;
18. WO 96/31622;
19. WO 97/10365;
20. WO 97/27317;
21. EP 373 203;
22. EP 785 280;
23. Louis S, Braudeau C, Giral M, Dupont A, Moizant F, Robillard N, Moreau A, Soulillou JP, Brouard S. 2006. Contrasting CD25hiCD4+T cells/FOXP3 patterns in chronic rejection and operational drug-free tolerance. 81(3):398-407.
24. WO 02/084567;
25. Roussey-Kesler G, Giral M, Moreau A, Subra JF, Legendre C, Noel C, Pillebout E, Brouard S, Soulillou JP. 2006. Clinical operational tolerance after kidney transplantation. Am J Transplant. 6(4):736-46.
26. Eisen, M. B., Spellman, P. T., Brown, P. O. & Botstein, D. 1998 Cluster analysis and display of genome-wide expression patterns. Proc Natl Acad Sci 95 (25): 14863-8.
27. Tibshirani, R., Hastie, T., Narasimhan, B. & Chu, G. 2002. Diagnosis of multiple cancer types by shrunken centroids of gene expression. Proc Natl Acad Sci U S A 99(10): 6567-72.

## Claims

1. Method for the in vitro diagnosis of a graft tolerant or non-tolerant phenotype, comprising:
(a) determining from a grafted subject biological sample an expression profile comprising TMTC3 gene,
(b) comparing the obtained expression profile with at least one reference expression profile, and
(c) determining the graft tolerant or graft non-tolerant phenotype from said comparison,
wherein said method does not comprise determining an expression profile comprising, in addition to TMTC3, the following 7 genes: BUB1B, CDC2, CHEK1, MS4A1, RAB30, RHOH, and SYNGR3.

2. The method of claim 1, wherein said expression profile consists of TMTC3 gene.

3. The method of claim 1 or claim 2, wherein the obtained expression profile is compared to at least one reference expression tolerant and/or not tolerant profile in step (b).

4. The method of any of claims 1-3, further comprising, if said subject is diagnosed as a graft non-tolerant subject, diagnosing from the expression profile if said subject is developing chronic rejection.

5. The method of any of claims 1-3, further comprising, if said subject is diagnosed as a graft non-tolerant subject, diagnosing from the expression profile if said subject is developing acute rejection.

6. The method of any of claims 1-5, wherein the expression profile is determined by measuring the amount of nucleic acid transcripts of said gene(s).

7. The method of claim 6, wherein the expression profile is determined using quantitative PCR or an oligonucleotide microarray comprising an oligonucleotide specific for TMTC3 gene1.

8. The method of any of claims 1-5, wherein the expression profile is determined using a genomic microarray or a proteic microarray.

9. The method according to any of claims 1-8, wherein said biological sample is a blood sample.

10. The method according to any of claims 1-9, wherein said subject is a kidney transplanted subject.

11. The method according to any of claims 1-10, further comprising determining at least one additional parameter selected from standard biological parameters specific for said subject grafted organ type, phenotypic analyses of peripheral blood mononuclear cells (PBMC), and qualitative and/or quantitative analysis of PBMC immune repertoire.

12. The method according to any of claims 1-11, further comprising between steps (b) and (c) the steps of:
(b1) obtaining from a grafted subject biological sample an expression profile comprising at least one gene from Table 2,
(b2) comparing the obtained expression profile with at least one reference expression profile, and
wherein in step (c), the graft tolerant or graft non-tolerant phenotype is determined from the comparison of both step (b1) and step (b2).
